# EUROPEAN PATENT APPLICATION

(11) **EP 2 100 899 A2**
(43) Date of publication of application: **16.09.2009**
(21) Application number: 09006228.2
(22) Date of filing: 19.10.2005
(51) Int. Cl.: C07K 5/087, C07K 5/08, A61K 38/06, G01N 33/573

(54) **Compounds for proteasome enzyme inhibition**

(30) Priority: 20.10.2004 US 620573 P; 26.04.2005 US 674834 P
(62) Divisional of application: 05812527.9
(71) Applicant: Proteolix, Inc., South San Francisco CA 94080 (US)
(72) Inventor: Bennett, Mark K., Moraga, CA 94456 (US); Buchholz, Tonia J., Ann Arbor, MI 48103 (US); Demo, Susan, San Francisco, CA 94117 (US); Laidig, Guy J., Menlo Park, CA 94025 (US); Lewis, Evan R., Pacifica, CA 94044 (US); Smyth, Mark S., Foster City, CA 94404 (US)
(74) Representative: Vossius & Partner

(57) **Abstract**

The present invention relates to a method for determining the activity of a proteasome inhibitor, comprising (a) obtaining a biological sample that has been treated with a proteasome inhibitor; (b) separating inhibitor-bound proteasome subunits from unbound proteasome subunits; (c) determining the amount of inhibitor-bound proteasome subunits, unbound proteasome subunits, or both, wherein a change in the amount of inhibitor bound proteasome subunits is indicative of proteasome inhibitor activity.

## Description

### Cross-Reference to Related Applications

This application claims the benefit of U.S. Provisional Application No. 60/620,573, filed October 20, 2004, and U.S. Provisional Application No. 60/674,834, filed April 26, 2005, the specifications of which are hereby incorporated by reference in their entirety.

### Technical Field

This invention relates to compounds and methods for enzyme inhibition. In particular, the invention relates to therapeutic methods based on enzyme inhibition.

### Background of the Invention

In eukaryotes, protein degradation is predominately mediated through the ubiquitin pathway in which proteins targeted for destruction are ligated to the 76 amino acid polypeptide ubiquitin. Once targeted, ubiquitinated proteins then serve as substrates for the 26S proteasome, a multicatalytic protease, which cleaves proteins into short peptides through the action of its three major proteolytic activities. While having a general function in intracellular protein turnover, proteasome-mediated degradation also plays a key role in many processes such as major histocompatibility complex (MHC) class I presentation, apoptosis, cell division, and NF-κB activation.

The 20S proteasome is a 700 kDa cylindrical-shaped multicatalytic protease complex comprised of 28 subunits organized into four rings that plays important roles in cell growth regulation, major histocompatibility complex class I presentation, apoptosis, antigen processing, NF-κB activation, and transduction of pro-inflammatory signals. In yeast and other eukaryotes, 7 different α subunits form the outer rings and 7 different β subunits comprise the inner rings. The α subunits serve as binding sites for the 19S (PA700) and 11S (PA28) regulatory complexes, as well as a physical barrier for the inner proteolytic chamber formed by the two β subunit rings. Thus, *in vivo,* the proteasome is believed to exist as a 26S particle ("the 26S proteasome"). *In vino* experiments have shown that inhibition of the 20S form of the proteasome can be readily correlated to inhibition of 26S proteasome. Cleavage of amino-terminal prosequences of β subunits during particle formation expose amino-terminal threonine residues, which serve as the catalytic nucleophiles. The subunits responsible for catalytic activity in proteasome thus possess an amino terminal nucleophilic residue, and these subunits belong to the family of N-terminal nucleophile (Ntn) hydrolases (where the nucleophilic N-terminal residue is, for example, Cys, Ser, Thr, and other nucleophilic moieties). This family includes, for example, penicillin G acylase (PGA), penicillin V acylase (PVA), glutamine PRPP amidotransferase (GAT), and bacterial glycosylasparaginase. In addition to the ubiquitously expressed β subunits, higher vertebrates also possess three γ-interferon-inducible β subunits (LMP7, LMP2 and MECLI), which replace their normal counterparts, X, Y and Z respectively, thus altering the catalytic activities of the proteasome. The term immunoproteasome refers to when all three interferon inducible β subunits are present. Through the use of different peptide substrates, three major proteolytic activities have been defined for the eukaryote 20S proteasome: chymotrypsin-like activity (CT-L), which cleaves after large hydrophobic residues; trypsin-like activity (T-L), which cleaves after basic residues; and peptidylglutamyl peptide hydrolyzing activity (PGPH), which cleaves after acidic residues. Two additional less characterized activities have also been ascribed to the proteasome: BrAAP activity, which cleaves after branched-chain amino acids; and SNAAP activity, which cleaves after small neutral amino acids. The major proteasome proteolytic activities appear to be contributed by different catalytic sites, since inhibitors, point mutations in β subunits and the exchange of γ interferon-inducing β subunits alter these activities to various degrees.

There are several examples of small molecules which have been used to inhibit proteasome activity; however, these compounds generally lack the specificity, stability, or potency necessary to explore and exploit the roles of the proteasome at the cellular and molecular level. Therefore, the synthesis of small molecule inhibitor(s) with increased site specificity, improved stability and solubility, and increased potency are needed to allow the exploration of the roles of the proteasome at the cellular and molecular level.

### Summary of the Invention

The invention relates to compounds known as peptide α',β'-epoxides and peptide α',β'-aziridines. The parent molecules are understood to bind efficiently, irreversibly and selectively to N-terminal nucleophile (Ntn) hydrolases, and can specifically inhibit particular activities of enzymes having multiple catalytic activity.

Once thought merely to dispose of denatured and misfolded proteins, the proteasome is now recognized as constituting proteolytic machinery that regulates the levels of diverse intracellular proteins through their degradation in a signal-dependent manner. Hence, there is great interest in identifying reagents that can specifically perturb the activities of the proteasome and other Ntn hydrolases and thereby be used as probes to study the role of these enzymes in biological processes. Compounds that target the Ntn hydrolases are herein described, synthesized and investigated. Peptide epoxides and peptide aziridines that can potently, selectively, and irreversibly inhibit particular proteasome activities are disclosed and claimed.

Particular peptide epoxides and peptide aziridines modify three catalytic subunits of the 20S proteasome resulting in inhibition primarily of the chymotrypsin-like activity. The peptide epoxides and peptide aziridines described herein are predicted to not substantially inhibit non-proteasomal proteases such as trypsin, chymotrypsin, cathepsin B, papain, and calpain at concentrations up to 50 µM. At higher concentrations, inhibition would be competitive and not irreversible, since the inhibitor merely competes with the substrate.

In one aspect, the invention provides inhibitors comprising a heteroatom-containing three-membered ring. These inhibitors can inhibit catalytic activity of N-terminal nucleophile hydrolase enzymes (for example, the 20S proteasome, or the 26S proteasome) when said inhibitor is present at concentrations below about 50 µM, and do not inhibit catalytic activity of non-proteasomal proteases when the inhibitor is present at concentrations below about 50 µM. Regarding the 20S proteasome, particular hydrolase inhibitors inhibit chymotrypsin-like activity of the 20S proteasome when the inhibitor is present at concentrations below about 5 µM. The hydrolase inhibitor can be, for example, a peptide α',β'-epoxy ketone or α',β'-aziridine ketone, and the peptide can be a tetrapeptide. The tetrapeptide can include branched or unbranched side chains such as hydrogen, C₁₋₆alkyl, C₁₋₆ hydroxyalkyl, C₁₋₆alkoxyalkyl, aryl, and C₁₋₆aralkyl, C₁₋₆alkylamide, C₁-₆alkylamine, C₁-₆carboxylic acid, C₁-₆carboxyl ester, C₁-₆alkylthiol, or C₁₋₆alkylthioether, for example isobutyl, 1-naphthyl, phenylmethyl, and 2-phenylethyl. The α'-carbon of the α',β'-epoxy ketone or α',β'-aziridine ketone can be a chiral carbon atom, such as an (R) or β configured carbon, as these are defined herein.

Another aspect of invention relates to pharmacodynamic assays, comprising administering a tagged proteasome inhibitor as set forth herein, e.g., wherein the tag is selected from a fluorescent moiety, a radioactive moiety, biotin, and a moiety that selectively binds to an antibody. This assay may be used to identify and characterize the binding characteristics of potential proteasome inhibitors.

In another aspect, the invention provides methods for determining the activity of a proteasome inhibitor. The assay involves contacting a sample with a tagged inhibitor and determining the amount of inhibitor-bound and/or unbound proteasome subunits. A tagged inhibitor as disclosed herein may be used to facilitate the determination of the amount of inhibitor-bound and/or unbound proteasome subunits, for example, using fluorescence polarization, detection of a radioactive signal, or detection of an affinity tag. In an exemplary embodiment, an assay involves separation of inhibitor-bound proteasome subunits from unbound subunits and determination of the amount of inhibitor-bound and/or unbound subunits. An affinity tagged proteasome inhibitor as set forth herein, may be used to facilitate separation of inhibitor-bound from unbound proteasome subunits. In an exemplary embodiment, an antibody specific for a proteasome subunit may be used to determine the amount of inhibitor-bound and/or unbound subunits. Use of a subunit-specific antibody permits differentiation of inhibition of the constitutive proteasome and the immunoproteasome.

In another aspect, the invention provides pharmaceutical compositions, including a pharmaceutically acceptable carrier, and a pharmaceutically effective amount of the hydrolase inhibitor, which ameliorates the effects of neurotoxic/neurodegenerative diseases (such as A1zheimer's disease), muscle-wasting diseases, proliferative diseases, cancer, chronic infectious diseases, fever, muscle disuse, denervation, nerve injury, immune related condition and fasting, among others.

In another aspect, the invention provides anti-inflammatory compositions.

In another aspect, the invention provides methods for the following: inhibiting or reducing HIV infection in a subject; affecting the level of viral gene expression in a subject; altering the variety of antigenic peptides produced by the proteasome in an organism; determining whether a cellular, developmental, or physiological process or output in an organism is regulated by the proteolytic activity of a particular Ntn hydrolase; treating Alzheimer's disease in a subject; treating ischemic conditions including macular degeneration; treating grafting rejection; treating septic shock; treating conditions associated with acidosis, macular degeneration, pulmonary conditions such as COPD and IPF, fibrotic diseases, and bone and hair growth; reducing the rate of muscle protein degradation in a cell; reducing the rate of intracellular protein degradation in a cell; reducing the rate of p53 protein degradation in a cell; inhibiting the growth of p53-related cancers in a subject; inhibiting antigen presentation in a cell; suppressing the immune system of a subject; inhibiting IκB-α degradation in an organism; reducing the content of NF-κB in a cell, muscle, organ or subject; affecting cyclin-dependent eukaryotic cell cycles; treating proliferative disease in a subject; affecting proteasome-dependent regulation of oncoproteins in a cell; treating cancer growth in a subject; treating p53-related apoptosis in a subject; and screening proteins processed by N-terminal nucleophile hydrolases in a cell. Each of these methods involves administering or contacting an effective amount of a composition comprising the hydrolase inhibitors disclosed herein, to a subject, a cell, a tissue, an organ, or an organism.

Other features and advantages of the invention will be apparent from the following detailed description, and from the claims.

### Brief Description of the Figures

Figure 1 shows the inhibitory activity of a fluorescein-tagged inhibitor (Inhibitor compared to an untagged inhibitor (YU101) *in vitro* with purified human 20S proteasome. Proteasome activity was measured via LLVY-AMC cleavage. Fluorescence was measured using the excitation wavelength of 340 nm and an emission wavelength of 465 nm. The assay was carried out in 20 mM Tris, pH 8.0, 0.5 mM EDTA, 0.03% SDS buffer with 5% DMSO.
Figure 2 shows the fluorescein polarization after incubating 2 nM of Inhibitor **1** with a serial dilution of purified human 20S proteasome (from 400 pM to 40 nM) in a 20 mM Tris, pH 8.0, 0.5 mM EDTA, 0.03% SDS buffer. Fluorescence was measured using the excitation wavelength of 485 nm and an emission wavelength of 535 nm.
Figure 3 shows the determination of drug occupancy by flow cytometry in HT29 cells using Inhibitor **2** with or without pretreatment with YU101.
Figure 4 shows an anti-beta5 Western blot for human 20S and whole blood lysate, demonstrating that streptavidin induces a shift in the electrophoretic mobility of the β5 subunit following treatment with Inhibitor **3**.
Figure 5 shows the correlation of the streptavidin gel shift assay with chymotryptic inhibition (Inhibitor **4** dose response) using either human 20S or whole blood treated with Inhibitor **3**.
Figure 6 shows a comparison of a β5 ELISA assay and a chymotryptic enzymatic assay (using the LLVY-AMC substrate peptide) in a human whole blood sample treated *ex vivo* with an irreversible peptide epoxyketone inhibitor.
Figure 7 shows a comparison of Inhibitor **3** (3) binding to the constitutive proteasome (β5) in whole blood and the immunoproteasome (LMP7) in PBMC.
Figure 8 shows a utility of the ELISA format assay using inhibitor **5** to monitor occupancy of multiple constitutive (β5, β1, β2) and immunoproteasome (LMP7, LMP2) active sites in extracts of 8226 multiple myeloma cells treated with either Inhibitor 4 or PS-341.

### Detailed Description of the Invention

The invention involves compounds useful as enzyme inhibitors. These compounds are generally useful as inhibitors of enzymes having a nucleophilic group at the N-terminus. For example, activities of enzymes or enzyme subunits having N-terminal amino acids with nucleophiles in their side chains, such as threonine, serine, or cysteine can be successfully inhibited by the enzyme inhibitors described herein. Activities of enzymes or enzyme subunits having non-amino acid nucleophilic groups at their N-termini, such as, for example, protecting groups or carbohydrates, can also be successfully inhibited by the enzyme inhibitors described herein.

While not bound by any particular theory of operation, it is believed that such N-terminal nucleophiles ofNtn form covalent adducts with the epoxide functional group of the enzyme inhibitors described herein. For example, in the β5/Pre2 subunit of 20S proteasome, the N-terminal threonine is believed to irreversibly form a morpholino or piperazino adduct upon reaction with a peptide epoxide or aziridine such as those described below. Such adduct formation would involve ring-opening cleavage of the epoxide or aziridine.

In embodiments including such groups bonded to α' carbons, the stereochemistry of the α'-carbon (that carbon forming a part of the epoxide or aziridine ring) can be (R) or (S). The invention is based, in part, on the structure-function information disclosed herein, which suggests the following preferred stereochemical relationships. Note that a preferred compound may have a number of stereocenters having the indicated up-down (or β-α, where β as drawn herein is above the plane of the page) or (R)-(S) relationship (that is, it is not required that every stereocenter in the compound conform to the preferences stated). In some preferred embodiments, the stereochemistry of the α' carbon is (R), that is, the X atom is β, or above the plane of the molecule.

Regarding the stereochemistry, the Cahn-Ingold-Prelog rules for determining absolute stereochemistry are followed. These rules are described, for example, in Organic Chemistry, Fox and Whitesell; Jones and Bartlett Publishers, Boston, MA (1994); Section 5-6, pp 177-178, which section is hereby incorporated by reference. Peptides can have a repeating backbone structure with side chains extending from the backbone units. Generally, each backbone unit has a side chain associated with it, although in some cases, the side chain is a hydrogen atom. In other embodiments, not every backbone unit has an associated side chain. Peptides useful in peptide epoxides or peptide aziridines have two or more backbone units. In some embodiments useful for inhibiting chymotrypsin-like (CT-L) activity of the proteasome, between four and eight backbone units are present, and in some preferred embodiments for CT-L inhibition, between four and six backbone units are present. In other embodiments useful for inhibiting the PGPH activity of the proteasome, between two and eight backbone units are present, and in some preferred embodiments for PGPH inhibition, between three and six backbone units are present.

The side chains extending from the backbone units can include natural aliphatic or aromatic amino acid side chains, such as hydrogen (glycine), methyl (alanine), isopropyl (valine), *sec-*butyl (isoleucine), isobutyl (leucine), phenylmethyl (phenylalanine), and the side chain constituting the amino acid proline. The side chains can also be other branched or unbranched aliphatic or aromatic groups such as ethyl, *n*-propyl, *n*-butyl, *t*-butyl, and aryl substituted derivatives such as 1-phenylethyl, 2-phenylethyl, (1-naphthyl)methyl, (2-naphthyl)methyl, 1-(1-naphthyl)ethyl, 1-(2-naphthyl)ethyl, 2-(1-naphthyl)ethyl, 2-(2-naphthyl)ethyl, and similar compounds. The aryl groups can be further substituted with branched or unbranched C₁₋₆alkyl groups, or substituted alkyl groups, acetyl and the like, or further aryl groups, or substituted aryl groups, such as benzoyl and the like. Heteroaryl groups can also be used as side chain substituents. Heteroaryl groups include nitrogen-, oxygen-, and sulfur-containing aryl groups such as thienyl, benzothienyl, naphthothienyl, thianthrenyl, furyl, pyranyl, isobenzofuranyl, chromenyl, pyrrolyl, imidazolyl, pyrazolyl, pyridyl, pyrazinyl, indolyl, purinyl, quinolyl, and the like.

In some embodiments, polar or charged residues can be introduced into the peptide epoxides or peptide aziridines. For example, naturally occurring amino acids such as hydroxy-containing (Thr, Tyr, Ser) or sulfur-containing (Met, Cys) can be introduced, as well as non-essential amino acids, for example, taurine, carnitine, citrulline, cystine, ornithine, norleucine and others. Non-naturally occurring side chain substituents with charged or polar moieties can also be included, such as, for example, C₁₋₆alkyl chains or C₆₋₁₂aryl groups with one or more hydroxy, short chain alkoxy, sulfide, thio, carboxyl, ester, phospho, amido or amino groups, or such substituents substituted with one or more halogen atoms. In some preferred embodiments, there is at least one aryl group present in a side chain of the peptide moiety.

In some embodiments, the backbone units are amide units [-NH-CHR-C(=O)-], in which R is the side chain. Such a designation does not exclude the naturally occurring amino acid proline, or other non-naturally occurring cyclic secondary amino acids, which will be recognized by those of skill in the art.

In other embodiments, the backbone units are N-alkylated amide units (for example, N-methyl and the like), olefinic analogs (in which one or more amide bonds are replaced by olefinic bonds), tetrazole analogs (in which a tetrazole ring imposes a cis-configuration on the backbone), or combinations of such backbone linkages. In still other embodiments, the amino acid α-carbon is modified by α-alkyl substitution, for example, aminoisobutyric acid. In some further embodiments, side chains are locally modified, for example, by ΔE or ΔZ dehydro modification, in which a double bond is present between the α and β atoms of the side chain, or for example by ΔE or ΔZ cyclopropyl modification, in which a cyclopropyl group is present between the α and β atoms of the side chain. In still further embodiments employing amino acid groups, D-amino acids can be used. Further embodiments can include side chain-to-backbone cyclization, disulfide bond formation, lactam formation, azo linkage, and other modifications discussed in "Peptides and Mimics, Design of Conformationally Constrained" by Hruby and Boteju, in "Molecular Biology and Biotechnology: A Comprehensive Desk Reference", ed. Robert A. Meyers, VCH Publishers (1995), pp. 658-664, which is hereby incorporated by reference.

In certain embodiments, the enzyme inhibitors have a structure of formula I or a pharmaceutically acceptable salt thereof, 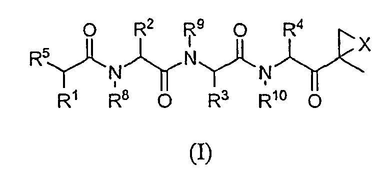
- X: is selected from O, NH, and N-C₁₋₆alkyl, preferably O;
- R¹,R², R³, and R⁴: are each independently selected from C₁₋₆alkyl, C₁₋₆hydroxyalkyl, C₁₋₆alkoxyalkyl, aryl, and C₁₋₆aralkyl, any of which is optionally substituted with one or more of amide, amine, carboxylic acid (or a salt thereof), ester (including C₁₋₅alkyl ester and aryl ester), thiol, or thioether substituents;
- R⁵: is N(R⁶)R⁷;
- R⁶: is selected from hydrogen, OH, and C₁₋₆alkyl, preferably H or C₁₋₆alkyl;
- R⁷: comprises a detectable label, such as a fluorescent moiety, a chemiluminescent moiety, a paramagnetic contrast agent, a metal chelate, a radioactive isotope-containing moiety, biotin, or a moiety that selectively binds to an antibody;
- R⁸,R⁹, and R¹⁰: are independently selected from hydrogen and C₁₋₆alkyl, preferably R⁸, R⁹, and R¹⁰ are all hydrogen;
provided that when R³ is C₁₋₆hydroxyalkyl, then R⁶ is hydrogen.

In some embodiments, R¹, R², R³, and R⁴ are selected from C₁₋₆alkyl or C₁₋₆aralkyl. In preferred embodiments, R² and R⁴ are C₁₋₆alkyl and R¹ and R³ are C₁₋₆aralkyl. In the most preferred embodiment, R² and R⁴ are isobutyl, R¹ is 2-phenylethyl, and R³ is phenylmethyl.

In certain embodiments, R⁶ is selected from H or C₁₋₆alkyl. In certain preferred embodiments, R⁶ is H.

In certain embodiments, R⁷ comprises both a detectable moiety and a linker moiety that joins the detectable moiety to the nitrogen atom of R⁵. In certain such embodiments, such as when the detectable moiety is biotin, the linker moiety may be absent or may be from 1 to 6 atoms in length.

In certain embodiments, R⁷ comprises a covalently conjugated moiety selected from a fluorescent moiety, a radioactive isotope-containing moiety, biotin, and a moiety that selectively binds to an antibody.

In certain embodiments, R⁷ comprises a fluorescent moiety. In certain such embodiments, the fluorescent moiety is an amine-reactive dye that has been covalently attached to the inhibitor. In preferred such embodiments, the amine-reactive dye is selected from Alexa Fluor dyes, BODIPY dyes, Cascade Blue dyes, coumarin, digoxigenin, fluorescein, lissamine rhodamine B dyes, Oregon Green dyes, rhodamine 6G dyes, rhodamine green dyes, rhodamine red dyes, Tamra, tetramethylrhodamine, and Texas Red dyes. In certain preferred embodiments, R⁷ comprises a fluorescent moiety selected from fluorescein, tetramethylrhodamine, and Tamra.

There are generally four classes of commonly used dye reagents to label amines:
succinimidyl esters, isothiocyanates, sulfonyl chlorides, and tetrafluorophenyl esters. Generally succinimidyl esters and tetrafluorophenyl esters are preferred for conjugation to proteins and peptides since they form a stable amide bond between the dye and the protein. Useful reviews that provide information on the conjugation of an amine-reactive dye to a protein or peptide sequence can be found in Bioconjug. Chem. 3, 2 (1992) and Methods Mol. Biol. 45, 205 (1995), incorporated herein by reference in their entirety. Information on the purchase and use of amine-reactive dyes is also available from Molecular Probes, Inc.

In certain embodiments, R⁷ contains a radioactive moiety. In certain such embodiments, R⁷ comprises a moiety selected from C₁₋₆alkyl, C₁-₆hydroxyalkyl, C₁₋₆alkoxyalkyl, aryl, and C₁₋₆aralkyl, wherein R⁷ includes at least one radioactive label selected from ³H, ¹¹C, ¹⁴C, ¹³N, ¹⁵O, and ¹²⁵I. In preferred such embodiments, R⁷ comprises an amino acid or peptide moiety that includes at least one radioactive label selected from 3H,¹¹C,¹⁴C, ¹³N, ¹⁵O, and ¹²⁵I.

In certain embodiments, R⁷ comprises a covalently conjugated moiety that selectively binds to an antibody that specifically binds to a peptide. In preferred embodiments, the moiety is selected from FLAG™, HA, HIS, c-Myc, VSV-G, V5 and HSV.

Preparation of inhibitors where R⁷ comprises a moiety selected from FLAG^{™}, HA, HIS, c-Myc, VSV-G, V5 and HSV may be accomplished using standard peptide coupling chemistry.

In certain embodiments, R⁷ comprises biotin which may be covalently conjugated to the inhibitor using standard carboxylic acid/amine coupling chemistry.

In certain embodiments, a compound of formula I has the following stereochemistry:

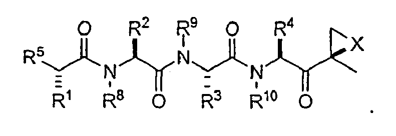

In certain preferred embodiments, the inhibitor has a structure of formula II or a pharmaceutically acceptable salt thereof, 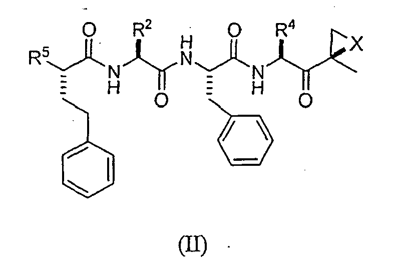
- X: is selected from O, NH, and N-C₁₋₆alkyl, preferably O;
- R²: and R⁴ are each independently selected from C₁₋₆alkyl, C₁₋₆hydroxyalkyl, C₁₋₆alkoxyalkyl, aryl, and C₁₋₆aralkyl, any of which is optionally substituted with one or more of amide, amine, carboxylic acid (or a salt thereof), ester (including C₁₋₅ alkyl ester and aryl ester), thiol, or thioether substituents;
- R⁵: is N(R⁶)R⁷;
- R⁶: is selected from hydrogen, OH, and C₁₋₆alkyl, preferably C₁₋₆alkyl;
- R⁷: comprises a detectable label, such as a fluorescent moiety, a chemiluminescent moiety, a paramagnetic contrast agent, a metal chelate, a radioactive isotope-containing moiety, biotin, or a moiety that selectively binds to an antibody.

### In certain embodiments, a compound of formula I is selected from

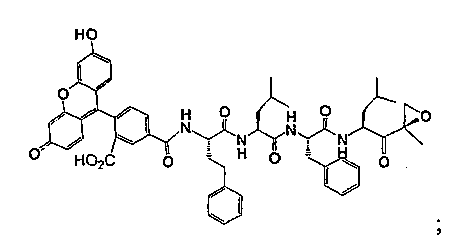

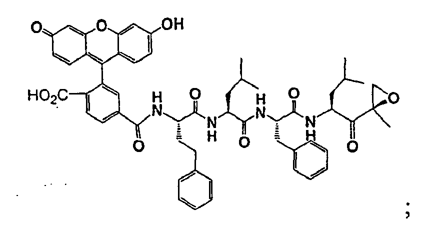

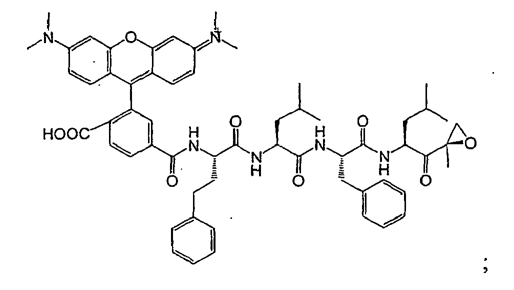

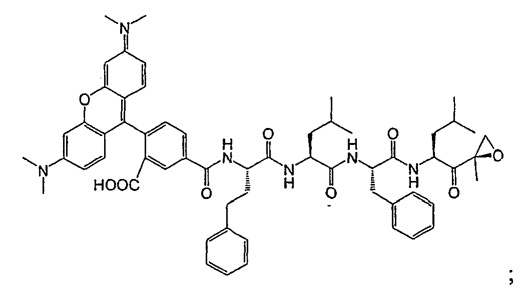

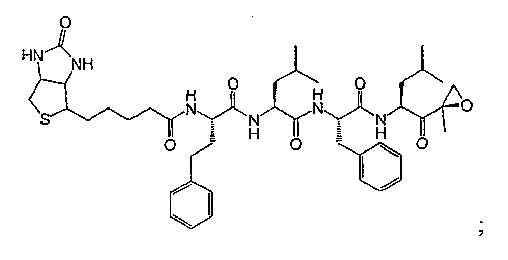

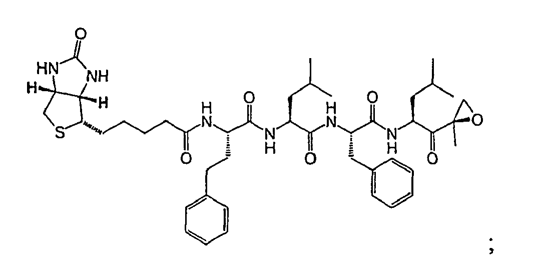

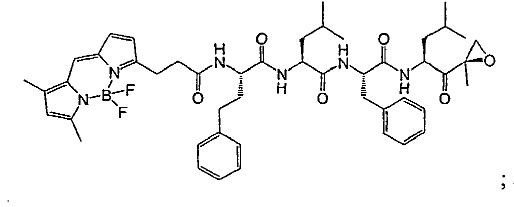

and

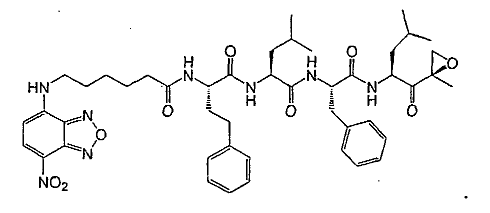

In certain embodiments, the enzyme inhibitors have a structure of formula III or a pharmaceutically acceptable salt thereof, 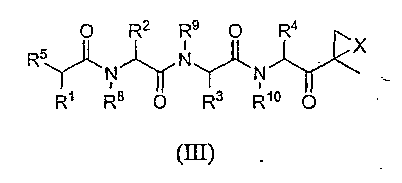
- X: is selected from O, NH, and N-C₁₋₆alkyl, preferably O;
- R¹,R², R³, and R⁴: are each independently selected from hydrogen, C₁₋₆alkyl, C₁₋₆hydroxyalkyl, C₁₋₆alkoxyalkyl, aryl, and C₁₋₆aralkyl, any of which is optionally substituted with one or more of amide, amine, carboxylic acid (or a salt thereof), ester (including C₁₋₅alkyl ester and aryl ester), thiol, or thioether substituents;
- R⁵: is N(R⁶)R⁷;
- R⁶: is selected from hydrogen, OH, and C₁₋₆alkyl, preferably H or C₁₋₆alkyl;

- R⁷: comprises a detectable label, such as a fluorescent moiety, a chemiluminescent moiety, a paramagnetic contrast agent, a metal chelate, a radioactive isotope-containing moiety, biotin, or a moiety that selectively binds to an antibody; and
- R⁸,R⁹, and R¹⁰: are independently selected from hydrogen and C₁₋₆alkyl; or R² and R⁸, R³ and R⁹, or R⁴ and R¹⁰ are together C₂₋₅alkyl, preferably C₃₋₄alkyl, thereby forming a ring;
provided that when R³ is C₁₋₆hydroxyalkyl, then R⁶ is hydrogen.

In certain embodiments, R¹, R², R³, and R⁴ are selected from hydrogen, C₁₋₆alkyl, and C₁₋₆aralkyl. In certain preferred embodiments, R¹ is hydrogen, R³ is selected from C₁₋₆alkyl and C₁₋₆aralkyl, and R⁴ is C₁₋₆alkyl. In certain preferred such embodiments, R¹ is hydrogen, R³ is selected from isobutyl and phenylmethyl, and R⁴ is isobutyl.

In certain embodiments, R² and R⁸ together are C₂₋₅alkyl, thereby forming a ring, preferably R² and R⁸ together are C₃alkyl, thereby forming a 5-membered ring. In certain alternative embodiments, R² is selected from C₁₋₆alkyl and C₁₋₆aralkyl, preferably C₁₋₆alkyl and R⁸ is hydrogen. In certain preferred such embodiments, R² is isobutyl.

In certain embodiments, R⁹ and R¹⁰ are independently selected from hydrogen and C₁₋₆alkyl, preferably R⁹ and R¹⁰ are both hydrogen.

In certain embodiments, R⁶ is selected from H or C₁₋₆alkyl. In certain preferred embodiments, R⁶ is H.

In certain embodiments, R⁷ comprises both a detectable moiety and a linker moiety that joins the detectable moiety to the nitrogen atom of R⁵. In certain such embodiments, such as when the detectable moiety is biotin, the linker moiety may be absent or may be from 1 to 6 atoms in length.

In certain embodiments, R⁷ comprises a covalently conjugated moiety selected from a fluorescent moiety, a radioactive isotope-containing moiety, biotin, and a moiety that selectively binds to an antibody.

In certain embodiments, R⁷ comprises a fluorescent moiety. In certain such embodiments, the fluorescent moiety is an amine-reactive dye that has been covalently attached to the inhibitor. In preferred such embodiments, the amine-reactive dye is selected from Alexa Fluor dyes, BODIPIY dyes, Cascade Blue dyes, coumarin, digoxigenin, fluorescein, lissamine rhodamine B dyes, Oregon Green dyes, rhodamine 6G dyes, rhodamine green dyes, rhodamine red dyes, Tamra, tetramethylrhodamine, and Texas Red dyes. In certain preferred embodiments, R⁷ comprises a fluorescent moiety selected from fluorescein, tetramethylrhodamine, and Tamra.

There are generally four classes of commonly used dye reagents to label amines:
succinimidyl esters, isothiocyanates, sulfonyl chlorides, and tetrafluorophenyl esters. Generally succinimidyl esters and tetrafluorophenyl esters are preferred for conjugation to proteins and peptides since they form a stable amide bond between the dye and the protein. Useful reviews that provide information on the conjugation of an amine-reactive dye to a protein or peptide sequence can be found in Bioconjug. Chem. 3,2 (1992) and Methods Mol. Biol. 45, 205 (1995), incorporated herein by reference in their entirety. Information on the purchase and use of amine-reactive dyes is also available from Molecular Probes, Inc.

In certain embodiments, R⁷ contains a radioactive moiety. In certain such embodiments, R⁷ comprises a moiety selected from C₁₋₆alk-yl, C₁₋₆hydroxyalkyl, C₁₋₆alkoxyalkyl, aryl, and C₁₋₆aralkyl, wherein R⁷ includes at least one radioactive label selected from ³H,¹¹C,¹⁴C, ¹³N, ¹⁵O, and ¹²⁵I. In preferred such embodiments, R⁷ comprises an amino acid or peptide moiety that includes at least one radioactive label selected from ³H,¹¹C, ¹⁴C, ¹³N, ¹⁵O, and ¹²⁵I.

In certain embodiments, R⁷ comprises a covalently conjugated moiety that selectively binds to an antibody that specifically binds to a peptide. In preferred embodiments, the moiety is selected from FLAG™, HA, HIS, c-Myc, VSV-G, V5 and HSV.

Preparation of inhibitors where R⁷ comprises a moiety selected from FLAG™, HA, HIS, c-Myc, VSV-G, V5 and HSV may be accomplished using standard peptide coupling chemistry.

In certain embodiments, R⁷ comprises biotin which may be covalently conjugated to the inhibitor using standard carboxylic acid/amine coupling chemistry.

In certain preferred embodiments, the inhibitor has a structure of formula IV or a pharmaceutically acceptable salt thereof, 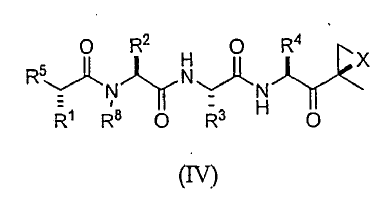
- X: is selected from O, NH, and N-C₁₋₆alkyl, preferably O;
- R¹,R² and R⁴: are each independently selected from hydrogen, C₁₋₆alkyl, C₁₋₆hydroxyalkyl, C₁₋₆alkoxyalkyl, aryl, and C₁₋₆aralkyl, any of which is optionally substituted with one or more of amide, amine, carboxylic acid (or a salt thereof), ester (including C₁₋₅alkyl ester and aryl ester), thiol, or thioether substituents;
- R⁵: is N(R⁶)R⁷;
- R⁶: is selected from hydrogen, OH, and C₁₋₆alkyl, preferably H or C₁₋₆alkyl;
- R⁷: comprises a detectable label, such as a fluorescent moiety, a chemiluminescent moiety, a paramagnetic contrast agent, a metal chelate, a radioactive isotope-containing moiety, biotin, or a moiety that selectively binds to an antibody; and
- R⁸: is selected from hydrogen and C₁₋₆alkyl; or R² and R⁸ are together C₂₋₃alkyl, thereby forming a ring.

In certain embodiments, a compound of formula III is selected from 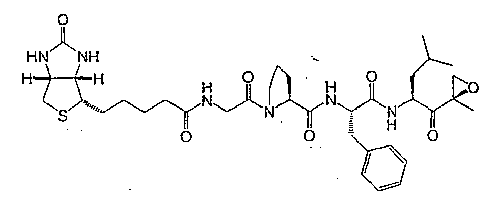 and

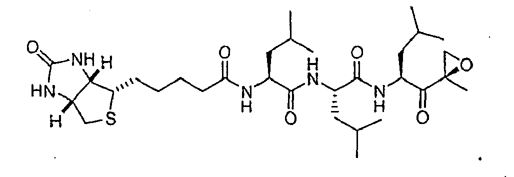

One aspect of the invention relates to a medical device including composition disclosed herein that include an inhibitor having a structure of any one of formulae I to IV. In one embodiment, the composition is incorporated within a medical device. In certain embodiments, the medical device is a gel comprising a polymer matrix or ceramic matrix and an inhibitor. Said polymer can be either naturally occurring or synthetic. In another embodiment, said gel serves as a drug depot, an adhesive, a suture, a barrier or a sealant.

Another aspect of the invention relates to a medical device comprising a substrate having a surface onto which an inhibitor having a structure of any one of formulae I to IV is disposed. In one embodiment, the inhibitor is directly disposed on a medical device.
In another embodiment, a coating is so disposed, the coating comprising a polymer matrix or ceramic matrix with an inhibitor having a structure of any one of formulae I to IV dispersed or dissolved therein.

In one embodiment, the medical device is a coronary, vascular, peripheral, or biliary stent. More particularly, the stent of the present invention is an expandable stent. When coated with a matrix containing an inhibitor having a structure any one of formulate I to IV, the matrix is flexible to accommodate compressed and expanded states of such an expandable stent. In another embodiment of this invention, the stent has at least a portion which is insertable or implantable into the body of a patient, wherein the portion has a surface which is adapted for exposure to body tissue and wherein at least a part of the surface is coated with an inhibitor having a structure of any one of formulae I to IV, or a coating comprising a matrix having an inhibitor having a structure of any one of formulae I to IV is dispersed or dissolved therein. An example of a suitable stent is disclosed in U.S. Pat. No. 4,733,665, which is incorporated herein by reference in its entirety.

In another embodiment, the medical device of the present invention is a surgical implement such as a vascular implant, an intraluminal device, surgical sealant or a vascular support. More particularly, the medical device of the present invention is a catheter, an implantable vascular access port, a central venous catheter, an arterial catheter, a vascular graft, an intraaortic balloon pump, a suture, a ventricular assist pump, a drug-eluting barrier, an adhesive, a vascular wrap, an extra/perivascular support, a blood filter, or a filter adapted for deployment in a blood vessel, coated with an inhibitor having a structure of any one of formulae I to IV either directly or by a matrix containing an inhibitor having a structure of any one of formulae I to IV.

In certain embodiments, the intraluminal medical device is coated with an inhibitor having a structure of any one of formulae I to IV or a coating comprising biologically tolerated matrix and an inhibitor having a structure of any one of formulae I to IV dispersed in the polymer, said device having an interior surface and an exterior surface, having the coating applied to at least a part of the interior surface, the exterior surface, or both.

In certain embodiments, the medical device may be useful to prevent restenosis after angioplasty. The medical device may also be useful for the treatment of various diseases and conditions by providing localized administration of an inhibitor having a structure of any one of formulae I to IV. Such diseases and conditions include restenosis, inflammation, rheumatoid arthritis, tissue injury due to inflammation, hyperproliferative diseases, severe or arthritic psoriasis, muscle-wasting diseases, chronic infectious diseases, abnormal immune response, conditions involving vulnerable plaques, injuries related to ischemic conditions, and viral infection and proliferation. Examples of diseases and conditions that are subject to a treatment including the drug coated medical devices of the present invention include atherosclerosis, acute coronary syndrome, Alzheimer's disease, cancer, fever, muscle disuse (atrophy), denervation, vascular occlusions, stroke, HIV infection, nerve injury, renal failure associated with acidosis, and hepatic failure. See, e.g., United States Patent No. 5,340,736 and United Stated Patent Application No. 11/106879.

The term "C_{x-y}alkyl" refers to substituted or unsubstituted saturated hydrocarbon groups, including straight-chain alkyl and branched-chain alkyl groups that contain from x to y carbons in the chain, including haloalkyl groups such as trifluoromethyl and 2,2,2-tirfluoroethyl, etc. C₀ alkyl indicates a hydrogen where the group is in a terminal position, a bond if internal. The terms "C_{2-y}alkenyl" and "C_{2-y}alkynyl" refer to substituted or unsubstituted unsaturated aliphatic groups analogous in length and possible substitution to the alkyls described above, but that contain at least one double or triple bond respectively.

The term "alkoxy" refers to an alkyl group having an oxygen attached thereto. Representative alkoxy groups include methoxy, ethoxy, propoxy, tert-butoxy and the like. An "ether" is two hydrocarbons covalently linked by an oxygen. Accordingly, the substituent of an alkyl that renders that alkyl an ether is or resembles an alkoxy.

The term "C₁₋₆alkoxyalkyl" refers to a C₁₋₆alkyl group substituted with an alkoxy group, thereby forming an ether.

The term "C₁₋₆aralkyl", as used herein, refers to a C₁₋₆alkyl group substituted with an aryl group.

The terms "amine" and "amino" are art-recognized and refer to both unsubstituted and substituted amines and salts thereof, e.g., a moiety that can be represented by the general formulae: 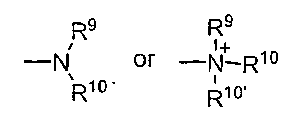 wherein R⁹, R¹⁰ and R¹⁰ each independently represent a hydrogen, an alkyl, an alkenyl, -(CH₂)ₘ-R⁸, or R⁹ and R¹⁰ taken together with the N atom to which they are attached complete a heterocycle having from 4 to 8 atoms in the ring structure; R⁸ represents an aryl, a cycloalkyl, a cycloalkenyl, a heterocyclyl or a polycyclyl; and m is zero or an integer from 1 to 8. In preferred embodiments, only one of R⁹ or R¹⁰ can be a carbonyl, e.g., R⁹, R¹⁰, and the nitrogen together do not form an imide. In even more preferred embodiments, R⁹ and R¹⁰ (and optionally R¹⁰) each independently represent a hydrogen, an alkyl, an alkenyl, or -(CH₂)ₘ-R⁸. In certain embodiments, an amino group is basic, meaning it has a pKₐ≥7.00. The protonated forms of these functional groups have pKₐs above 7.00.

The terms "amide" and "amido" are art-recognized as an amino-substituted carbonyl and includes a moiety that can be represented by the general formula: 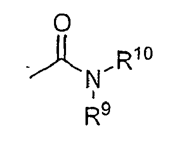 wherein R⁹, R¹⁰ are as defined above. Preferred embodiments of the amide will not include imides which may be unstable.

The term "aryl" as used herein includes 5-, 6-, and 7-membered substituted or unsubstituted single-ring aromatic groups in which each atom of the ring is carbon. The term "aryl" also includes polycyclic ring systems having two or more cyclic rings in which two or more carbons are common to two adjoining rings wherein at least one of the rings is aromatic, e.g., the other cyclic rings can be cycloalLyls, cycloalkenyls, cycloalkynyls, aryls, heteroaryls, and/or heterocyclyls. Aryl groups include benzene, naphthalene, phenanthrene, phenol, aniline, and the like.

The term "carbonyl" is art-recognized and includes such moieties as can be represented by the general formula: 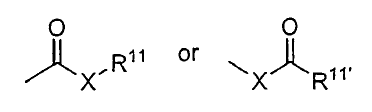 wherein X is a bond or represents an oxygen or a sulfur, and R¹¹ represents a hydrogen, an alkyl, an alkenyl, -(CH₂)ₘ-R⁸ or a pharmaceutically acceptable salt, R^{11'} represents a hydrogen, an alkyl, an alkenyl or -(CH₂)ₘ-R⁸, where m and R⁸ are as defined above. Where X is an oxygen and R¹¹ or R^{11'} is not hydrogen, the formula represents an "ester". Where X is an oxygen, and R¹¹ is a hydrogen, the formula represents a "carboxylic acid".

As used herein, "enzyme" can be any partially or wholly proteinaceous molecule which carries out a chemical reaction in a catalytic manner. Such enzymes can be native enzymes, fusion enzymes, proenzymes, apoenzymes, denatured enzymes, farnesylated enzymes, ubiquitinated enzymes, fatty acylated enzymes, gerangeranylated enzymes, GPI-linked enzymes, lipid-linked enzymes, prenylated enzymes, naturally-occurring or artificially-generated mutant enzymes, enzymes with side chain or backbone modifications, enzymes having leader sequences, and enzymes complexed with non-proteinaceous material, such as proteoglycans, proteoliposomes. Enzymes can be made by any means, including natural expression, promoted expression, cloning, various solution-based and solid-based peptide syntheses, and similar methods known to those of skill in the art.

The term "C₁₋₆hydroxyalkyl" refers to a C₁₋₆alkyl group substituted with a hydroxy group.

As used herein, the term "inhibitor" is meant to describe a compound that blocks or reduces an activity of an enzyme (for example, inhibition of proteolytic cleavage of standard fluorogenic peptide substrates such as suc-LLVY-AMC, Box-LLR-AMC and Z-LLE-AMC, inhibition of various catalytic activities of the 20S proteasome). An inhibitor can act with competitive, uncompetitive, or noncompetitive inhibition. An inhibitor can bind reversibly or irreversibly, and therefore the term includes compounds that are suicide substrates of an enzyme. An inhibitor can modify one or more sites on or near the active site of the enzyme, or it can cause a conformational change elsewhere on the enzyme.

The term "linker", as used herein, refers to a covalent linkage that comprises from 1-20 atoms in the chain that connects or links the two moieties at either end. Suitable linkers include one or more amino acid residues, alkyl chains (e.g., ethylene, propylene, etc., branched or unbranched), or similar chains in which some carbon atoms (preferably not two adjacent ones) have been replaced by heteroatoms, such as sulfur, oxygen, or nitrogen (substituted or unsubstituted). Linkers may be substituted or unsubstituted, and, although typically acyclic, may include a ring. Linkers may have groups at one or both termini that facilitate attachment and/or cleavage of the linkers, such as carbonyl, sulfonyl, and thiocarbonyl moieties. Thus, exemplary linkers include -(CH₂)₆-,-CH2C(O)-, -(CH₂)₂O(CH₂)₂-, -CH₂-(p-phenyl)-CH₂-, -C(O)N(Me)CH₂CH₂OC(O)-, - (CH₂)₂C(O)NH(CH₂)₂S(CH₂)₂N(Et), etc.

As used herein, the term "peptide" includes not only standard amide linkage with standard α-substituents, but commonly utilized peptidomimetics, other modified linkages, non-naturally occurring side chains, and side chain modifications, as detailed below.

The terms "polycyclyl" or "polycyclic" refer to two or more rings (e.g., cycloalkyls, cycloalkenyls, cycloalkynyls, aryls, heteroaryls, and/or heterocyclyls) in which two or more carbons are common to two adjoining rings, e.g., the rings are "fused rings". Each of the rings of the polycycle can be substituted or unsubstituted.

The term "preventing" is art-recognized, and when used in relation to a condition, such as a local recurrence (e.g., pain), a disease such as cancer, a syndrome complex such as heart failure or any other medical condition, is well understood in the art, and includes administration of a composition which reduces the frequency of, or delays the onset of, symptoms of a medical condition in a subject relative to a subject which does not receive the composition. Thus, prevention of cancer includes, for example, reducing the number of detectable cancerous growths in a population of patients receiving a prophylactic treatment relative to an untreated control population, and/or delaying the appearance of detectable cancerous growths in a treated population versus an untreated control population, e.g., by a statistically and/or clinically significant amount. Prevention of an infection includes, for example, reducing the number of diagnoses of the infection in a treated population versus an untreated control population, and/or delaying the onset of symptoms of the infection in a treated population versus an untreated control population. Prevention of pain includes, for example, reducing the magnitude of, or alternatively delaying, pain sensations experienced by subjects in a treated population versus an untreated control population.

The term "prophylactic or therapeutic" treatment is art-recognized and includes administration to the host of one or more of the subject compositions. If it is administered prior to clinical manifestation of the unwanted condition (e.g., disease or other unwanted state of the host animal) then the treatment is prophylactic, (i.e., it protects the host against developing the unwanted condition), whereas if it is administered after manifestation of the unwanted condition, the treatment is therapeutic, (i.e., it is intended to diminish, ameliorate, or stabilize the existing unwanted condition or side effects thereof).

The term "proteasome" as used herein is meant to include both immuno- and constitutive forms, unless otherwise specified.

The term "substituted" refers to moieties having substituents replacing a hydrogen on one or more carbons of the backbone. It will be understood that "substitution" or "substituted with" includes the implicit proviso that such substitution is in accordance with permitted valence of the substituted atom and the substituent, and that the substitution results in a stable compound, e.g., which does not spontaneously undergo transformation such as by rearrangement, cyclization, elimination, etc. As used herein, the term "substituted" is contemplated to include all permissible substituents of organic compounds. In a broad aspect, the permissible substituents include acyclic and cyclic, branched and unbranched, carbocyclic and heterocyclic, aromatic and non-aromatic substituents of organic compounds. The permissible substituents can be one or more and the same or different for appropriate organic compounds. For purposes of this invention, the heteroatoms such as nitrogen may have hydrogen substituents and/or any permissible substituents of organic compounds described herein which satisfy the valences of the heteroatoms. Substituents can include, for example, a halogen, a hydroxyl, a carbonyl (such as a carboxyl, an alkoxycarbonyl, a formyl, or an acyl), a thiocarbonyl (such as a thioester, a tlioacetate, or a thioformate), an alkoxyl, a phosphoryl, a phosphate, a phosphonate, a phosphinate, an amino, an amido, an amidine, an imine, a cyano, a nitro, an azido, a sulfhydryl, an alkylthio, a sulfate, a sulfonate, a sulfamoyl, a sulfonamido, a sulfonyl, a heterocyclyl, an aralkyl, or an aromatic or heteroaromatic moiety. It will be understood by those skilled in the art that the moieties substituted on the hydrocarbon chain can themselves be substituted, if appropriate.

A "therapeutically effective amount" of a compound with respect to the subject method of treatment, refers to an amount of the compound(s) in a preparation which, when administered as part of a desired dosage regimen (to a mammal, preferably a human) alleviates a symptom, ameliorates a condition, or slows the onset of disease conditions according to clinically acceptable standards for the disorder or condition to be treated or the cosmetic purpose, e.g., at a reasonable benefit/risk ratio applicable to any medical treatment

The term "thioether" refers to an alkyl group, as defined above, having a sulfur moiety attached thereto. In preferred embodiments, the "thioether" is represented by -S-alkyl. Representative thioether groups include methylthio, ethylthio, and the like.

As used herein, the term "treating" or "treatment" includes reversing, reducing, or arresting the symptoms, clinical signs, and underlying pathology of a condition in manner to improve or stabilize a subject's condition.

### Selectivity for 20S proteasome

The enzyme inhibitors disclosed herein are useful in part because they inhibit the action of the 20S proteasome. Additionally, unlike other 20S proteasome inhibitors, the compounds disclosed herein are highly selective toward the 20S proteasome, with respect to other protease enzymes. That is, the instant compounds show selectivities for the 20S proteasome over other proteases such as cathepsins, calpains, papain, chymotrypsin, trypsin, tripeptidyl pepsidase II. The selectivities of the enzyme inhibitors for 20S proteasome are such that at concentrations below about 50 µM, the enzyme inhibitors show inhibition of the catalytic activity of the 20S proteasome, while not showing inhibition of the catalytic activity of other proteases such as cathepsins, calpains, papain, chymotrypsin, trypsin, tripeptidyl pepsidase II. In preferred embodiments, the enzyme inhibitors show inhibition of the catalytic activity of the 20S proteasome at concentrations below about 10 µM, while not showing inhibition of the catalytic activity of other proteases at these concentrations. In even more preferred embodiments, the enzyme inhibitors show inhibition of the catalytic activity of the 20S proteasome at concentrations below about 1 µM, while not showing inhibition of the catalytic activity of other proteases at these concentrations. Enzyme kinetic assays are disclosed in U.S. application serial number 09/569748, Example 2 and Stein et al., Biochem. (1996), 35, 3899-3908.

### Selectivity for Chymotrypsin-Like Activity

Particular embodiments of the enzyme inhibiting compounds described herein are further useful because they can efficiently and selectively inhibit the chymotrypsin-like activity of the 20S proteasome, as compared to the trypsin-like, and PGPH activities. The chymotrypsin-like activity of 20S proteasome is characterized by cleavage of peptides in the immediate vicinity of large hydrophobic residues. In particular, the chymotrypsin-like activity of Ntn hydrolases can be determined by cleavage of a standard substrate. Examples of such substrates are known in the art. For example, a leucylleucylvalinyltyrosine derivative can be used. The enzyme kinetic assays are disclosed in U.S. application serial number 09/569748, Example 2 and Stein et al., Biochem. (1996), 35, 3899-3908.

### Selectivity for PGPH Activity

Particular embodiments of the enzyme inhibiting compounds described herein are further useful because they can efficiently and selectively inhibit the PGPH activity of the 20S proteasome, as compared to the chymotrypsin-like and trypsin-like activities upon hydrolysis to provide the inhibitor itself. The PGPH activity of 20S proteasome is characterized by cleavage of peptides in the immediate vicinity of acidic residues. In particular, the PGPH activity of Ntn hydrolases can be determined by cleavage of a standard substrate. Examples of such substrates are known in the art. For example, a leucylleucylglutamate derivative can be used.

### Uses of Enzyme Inhibitors

Using epoxomicin and its biotinylated affinity derivative, it has been shown that epoxomicin covalently binds the LMP7, X, Z, and MECLI catalytic β subunits of the 20S proteasome and selectively inhibits the three major 20S proteasome proteolytic activities at different rates. Both *in vitro* and *in vivo* data demonstrated that epoxomicin effectively inhibited NF-κB-mediated pro-inflammatory signaling. Given the unique specificity and potency, this natural product represents a novel class of irreversible inhibitors distinct from the reagents currently in use and, thus, might prove useful in *in vivo* and *in vitro* analyses of proteasome function.

The biological consequences of proteasome inhibition are numerous. At the cellular level, the accumulation ofpolyubiquitinated proteins, cell morphological changes, and apoptosis have been reported upon treatment of cells with various proteasome inhibitors. In analyses, it has been observed that p53 levels are stabilized over 50-fold by epoxomicin treatment. In addition, results showing an accumulation of polyubiquitinated proteins in epoxomicin-treated cells provide evidence that the proteasome is the target of epoxomicin. Proteasome inhibition has also been suggested as a possible antitumor therapeutic strategy. The fact that epoxomicin was initially identified in a screen for antitumor compounds validates the proteasome as an antitumor chemotherapeutic target. Moreover, in parallel with this study, another antitumor natural product was found, eponemycin, that also targets the proteasome, although, less potently.

Peptide epoxides and peptide aziridines can inhibit NF-κB activation, and stabilize p53 levels in cell culture. Since NF-κB is a key regulator of inflammation, it is an attractive target for anti-inflammatory therapeutic intervention. Thus, compounds of the invention may be useful for the treatment of conditions associated with inflammation, including, but not limited to COPD, psoriasis, bronchitis, emphysema, and cystic fibrosis.

The disclosed compounds can be used to treat conditions mediated directly by the proteolytic function of the proteasome such as muscle wasting, or mediated indirectly via proteins which are processed by the proteasome such as NF-κB. The proteasome participates in the rapid elimination and post-translational processing of proteins (e.g., enzymes) involved in cellular regulation (e.g., cell cycle, gene transcription, and metabolic pathways), intercellular communication, and the immune response (e.g., antigen presentation). Specific examples discussed below include β-amyloid protein and regulatory proteins such as cyclins and transcription factor NF-κB.

Another embodiment of the invention is the use of the compounds disclosed herein for the treatment of neurodegenerative diseases and conditions, including, but not limited to, stroke, ischemic damage to the nervous system, neural trauma (e.g., percussive brain damage, spinal cord injury, and traumatic damage to the nervous system), multiple sclerosis and other immune-mediated neuropathies (e.g., Guillain-Barre syndrome and its variants, acute motor axonal neuropathy, acute inflammatory demyelinating polyneuropathy, and Fisher Syndrome), HIV/AIDs dementia complex, axonomy, diabetic neuropathy, Parkinson's disease, Huntington's disease, multiple sclerosis, bacterial, parasitic, fungal, and viral meningitis, encephalitis, vascular dementia, multi-infarct dementia, Lewy body dementia, frontal lobe dementia such as Pick's disease, subcortical dementias (such as Huntington or progressive supranuclear palsy), focal cortical atrophy syndromes (such as primary aphasia), metabolic-toxic dementias (such as chronic hypothyroidism or B12 deficiency), and dementias caused by infections (such as syphilis or chronic meningitis).

Alzheimer's disease is characterized by extracellular deposits of β-amyloid protein β-AP) in senile plaques and cerebral vessels. β-AP is a peptide fragment of 39 to 42 amino acids derived from an amyloid protein precursor (APP). At least three isoforms of APP are known (695, 751, and 770 amino acids). Alternative splicing of mRNA generates the isoforms; normal processing affects a portion of the β-AP sequence, thereby preventing the generation of β-AP. It is believed that abnormal protein processing by the proteasome contributes to the abundance of β-AP in the Alzheimer brain. The APP-processing enzyme in rats contains about ten different subunits (22 kDa-32 kDa). The 25 kDa subunit has an N-terminal sequence of X-Gln-Asn-Pro-Met-X-Thr-Gly-Thr-Ser, which is identical to the β-subunit of human macropain (Kojima, S. et al., Fed. Eur. Biochem. Soc., (1992) 304:57-60). The APP-processing enzyme cleaves at the Gln¹⁵-Lys¹⁶ bond; in the presence of calcium ion, the enzyme also cleaves at the Met-¹ --Asp¹ bond, and the Asp¹ --Ala² bonds to release the extracellular domain of β-AP.
- One embodiment, therefore, is a method of treating Alzheimer's disease, including administering to a subject an effective amount of a compound (e.g., pharmaceutical composition) disclosed herein. Such treatment includes reducing the rate of β-AP processing, reducing the rate of β-AP plaque formation, reducing the rate of β-AP generation, and reducing the clinical signs of Alzheimer's disease.

Other embodiments of the invention relate to cachexia and muscle-wasting diseases. The proteasome degrades many proteins in maturing reticulocytes and growing fibroblasts. In cells deprived of insulin or serum, the rate of proteolysis nearly doubles. Inhibiting the proteasome reduces proteolysis, thereby reducing both muscle protein loss and the nitrogenous load on kidneys or liver. Proteasome inhibitors are useful for treating conditions such as cancer, chronic infectious diseases, fever, muscle disuse (atrophy) and denervation, nerve injury, fasting, renal failure associated with acidosis, and hepatic failure. See, e.g., Goldberg, U.S. Patent No. 5,340,736. Embodiments of the invention therefore encompass methods for: reducing the rate of muscle protein degradation in a cell; reducing the rate of intracellular protein degradation; reducing the rate of degradation of p53 protein in a cell; and inhibiting the growth of p53-related cancers. Each of these methods includes the step of contacting a cell (*in vivo* or *in vitro,* e.g., a muscle in a subject) with an effective amount of a compound (e.g., pharmaceutical composition) disclosed herein.

Fibrosis is the excessive and persistent formation of scar tissue resulting from the hyperproliferative growth of fibroblasts and is associated with activation of the TGF-β signaling pathway. Fibrosis involves extensive deposition of extracellular matrix and can occur within virtually any tissue or across several different tissues. Normally, the level of intracellular signaling protein (Smad) that activate transcription of target genes upon TGF-β stimulation is regulated by proteasome activity (Xu et al., 2000). However, accelerated degradation of the TGF-β signaling components has been observed in cancers and other hyperproliferative conditions. Thus, certain embodiments of the invention relate to a method for treating hyperproliferative conditions such as diabetic retinopathy, macular degeneration, diabetic nephropathy, glomerulosclerosis, IgA nephropathy, cirrhosis, biliary atresia, congestive heart failure, scleroderma, radiation-induced fibrosis, and lung fibrosis (idiopathic pulmonary fibrosis, collagen vascular disease, sarcoidosis, interstitial lung diseases and extrinsic lung disorders). The treatment of burn victims is often hampered by fibrosis, thus, an additional embodiment of the invention is the topical or systemic administration of the inhibitors to treat bums. Wound closure following surgery is often associated with disfiguring scars, which may be prevented by inhibition of fibrosis. Thus, in certain embodiments, the invention relates to a method for the prevention or reduction of scarring.

Another protein processed by the proteasome is NF-κB, a member of the Rel protein family. The Rel family of transcriptional activator proteins can be divided into two groups. The first group requires proteolytic processing, and includes p50 (NF-κB1, 105 kDa) and p52 (NF-κ2, 100 kDa). The second group does not require proteolytic processing, and includes p65 (ReIA, Rel (c-Rel), and ReIB). Both homo- and heterodimers can be formed by Rel family members; NF-κB, for example, is a p50-p65 heterodimer. After phosphorylation and ubiquitination of IκB and p¹⁰⁵, the two proteins are degraded and processed, respectively, to produce active NF-κB which translocates from the cytoplasm to the nucleus. Ubiquitinated p¹⁰⁵ is also processed by purified proteasomes (Palombella et al., Cell (1994) 78:773-785). Active NF-κB forms a stereospecific enhancer complex with other transcriptional activators and, e.g., HMG I(Y), inducing selective expression of a particular gene.

NF-κB regulates genes involved in the immune and inflammatory response, and mitotic events. For example, NF-κB is required for the expression of the immunoglobulin light chain κ gene, the IL-2 receptor α-chain gene, the class I major histocompatibility complex gene, and a number of cytokine genes encoding, for example, IL-2, IL-6, granulocyte colony-stimulating factor, and IFN-β (Palombella et al., Cell (1994) 78:773-785). Some embodiments of the invention include methods of affecting the level of expression of IL-2, MHC-I, EL-6, IFN-β or any of the other previously-mentioned proteins, each method including administering to a subject an effective amount of a compound disclosed herein. Complexes including p50 are rapid mediators of acute inflammatory and immune responses (Thanos, D. and Maniatis, T., Cell (1995) 80:529-532).

NF-κB also participates in the expression of the cell adhesion genes that encode E-selectin, P-selectin, ICAm, and VCAM-1 (Collins, T., Lab. Invest. (1993) 68:499-508). One embodiment of the invention is a method for inhibiting cell adhesion (e.g., cell adhesion mediated by E-selectin, P-selectin, ICAm, or VCAM-1), including contacting a cell with (or administering to a subject) an effective amount of a compound (or a pharmaceutical composition) disclosed herein.

Ischemia and reperfusion injury results in hypoxia, a condition in which there is a deficiency of oxygen reaching the tissues of the body. This condition causes increased degradation of Iκ-Bα, thereby resulting in the activation of NF-κB (Koong et al., 1994). It has been demonstrated that the severity of injury resulting in hypoxia can be reduced with the administration of a proteasome inhibitor (Gao et al., 2000; Bao et al., 2001; Pye et al., 2003). Therefore, certain embodiments of the invention relate to a method of treating an ischemic condition or reperfusion injury comprising administering to a subject in need of such treatment an effective amount of a compound disclosed herein. Examples of such conditions or injuries include, but are not limited to, acute coronary syndrome (vulnerable plaques), arterial occlusive disease (cardiac, cerebral, peripheral arterial and vascular occlusions), atherosclerosis (coronary sclerosis, coronary artery disease), infarctions, heart failure, pancreatitis, myocardial hypertrophy, stenosis, and restenosis.

NF*-κ*B also binds specifically to the HIV-enhancer/promoter. When compared to the Nef of mac23 9, the HIV regulatory protein Nef of pbj14 differs by two amino acids in the region which controls protein kinase binding. It is believed that the protein kinase signals the phosphorylation of I-κB, triggering IκB degradation through the ubiquitin-proteasome pathway. After degradation, NF-κB is released into the nucleus, thus enhancing the transcription of HIV -(Cohen, J., Science, (1995) 267:960). Two embodiments of the invention are a method for inhibiting or reducing HIV infection in a subject, and a method for decreasing the level of viral gene expression, each method including administering to the subject an effective amount of a compound disclosed herein.

Overproduction of lipopolysaccharide (LPS)-induced cytokines such as TNFα is considered to be central to the processes associated with septic shock. Furthermore, it is generally accepted that the first step in the activation of cells by LPS is the binding of LPS to specific membrane receptors. The α- and β-subunits of the 20S proteasome complex have been identified as LPS-binding proteins, suggesting that the LPS-induced signal transduction may be an important therapeutic target in the treatment or prevention of sepsis (Qureshi, N. et al., J. Immun. (2003) 171: 1515-1525). Therefore, in certain embodiments, compounds of the invention may be used for the inhibition of TNFα to prevent and/or treat septic shock.

Intracellular proteolysis generates small peptides for presentation to T-lymphocytes to induce MHC class I-mediated immune responses. The immune system screens for autologous cells that are virally infected or have undergone oncogenic transformation. One embodiment is a method for inhibiting antigen presentation in a cell, including exposing the cell to a compound described herein. A compound of the invention may be used to treat immune-related conditions such as allergy, asthma, organ/tissue rejection (graft-versus-host disease), and auto-immune diseases, including, but not limited to, lupus, rheumatoid arthritis, psoriasis, multiple sclerosis, and inflammatory bowel diseases (such as ulcerative colitis and Crohn's disease). Thus, a further embodiment is a method for suppressing the immune system of a subject including administering to the subject an effective amount of a compound described herein.

Another further embodiment is a method for altering the repertoire of antigenic peptides produced by the proteasome or other Ntn with multicatalytic activity. For example, if the PGPH activity of 20S proteasome is selectively inhibited, a different set of antigenic peptides will be produced by the proteasome and presented in MHC molecules on the surfaces of cells than would be produced and presented either without any enzyme inhibition, or with, for example, selective inhibition of chymotrypsin-like activity of the proteasome.

Certain proteasome inhibitors block both degradation and processing of ubiquitinated NF-κB *in vitro and in vivo.* Proteasome inhibitors also block IκB-A degradation and NF-κB activation (Palombella, et al. Cell (1994) 78:773-785; and Traenckner, et al., EMBO J. (1994) 13:5433-5441). One embodiment of the invention is a method for inhibiting IκB-α degradation, including contacting the cell with a compound described herein. A further embodiment is a method for reducing the cellular content of NF-κB in a cell, muscle, organ, or subject, including contacting the cell, muscle, organ, or subject with a compound described herein.

Other eukaryotic transcription factors that require proteolytic processing include the general transcription factor TFIIA, herpes simplex virus VP 16 accessory protein (host cell factor), virus-inducible IFN regulatory factor 2 protein, and the membrane-bound sterol regulatory element-binding protein 1.

Other embodiments of the invention are methods for affecting cyclin-dependent eukaryotic cell cycles, including exposing a cell (*in vitro* or *in vivo*) to a compound disclosed herein. Cyclins are proteins involved in cell cycle control. The proteasome participates in the degradation of cyclins. Examples of cyclins include mitotic cyclins, G1 cyclins, (cyclin B). Degradation of cyclins enables a cell to exit one cell cycle stage (e.g., mitosis) and enter another (e.g., division). It is believed all cyclins are associated with p34^{cdc2} protein kinase or related kinases. The proteolysis targeting signal is localized to amino acids 42-RAALGNISEN-50 (destruction box). There is evidence that cyclin is converted to a form vulnerable to a ubiquitin ligase or that a cyclin-specific ligase is activated during mitosis (Ciechanover, A., Cell, (1994) 79:13-21). Inhibition of the proteasome inhibits cyclin degradation, and therefore inhibits cell proliferation, for example, in cyclin-related cancers (Kumatori et al., Proc. Natl. Acad. Sci. USA (1990) 87:7071-7075). One embodiment of the invention is a method for treating a proliferative disease in a subject (e.g., cancer, psoriasis, or restenosis), including administering to the subject an effective amount of a compound disclosed herein. The invention.also encompasses a method for treating cyclin-related inflammation in a subject, including administering to a subject a therapeutically effective amount of a compound described herein.

Additional embodiments are methods for affecting the proteasome-dependent regulation of oncoproteins and methods of treating or inhibiting cancer growth, each method including.exposing a cell (*in vivo,* e.g., in a subject or *in vitro)* to a compound disclosed herein. HPV-16 and HPV-18-derived E6 proteins stimulate ATP-and ubiquitin-dependent conjugation and degradation of p53 in crude reticulocyte lysates. The recessive oncogene p53 has been shown to accumulate at the nonpermissive temperature in a cell line with a mutated thermolabile E1. Elevated levels of p53 may lead to apoptosis. Examples of proto-oncoproteins degraded by the ubiquitin system include c-Mos, c-Fos, and c-Jun. One embodiment is a method for treating p53-related apoptosis, including administering to a subject an effective amount of a compound disclosed herein.

In another embodiment, the disclosed compounds are useful for the treatment of a parasitic infection, such as infections caused by protozoan parasites. The proteasome of these parasites is considered to be involved primarily in cell differentiation and replication activities (Paugam et al., Trends Parasitol. 2003, 19(2): 55-59). Furthermore, entamoeba species have been shown to lose encystation capacity when exposed to proteasome inhibitors (Gonzales, et al., Arch. Med. Res. 1997, 28, Spec No: 139-140). In certain such embodiments, the disclosed compounds are useful for the treatment of parasitic infections in humans caused by a protozoan parasite selected from Plasmodium sps. (including P. falciparum, P. vivax, P. malariae, and P. ovale, which cause malaria), Trypanosoma sps. (including T. cruzi, which causes Chagas' disease, and T. brucei which causes African sleeping sickness), Leishmania sps. (including L. amazonesis, L. donovani, L. infantum, L. mexicana, etc.), Pneumocystis carinii (a protozoan known to cause pneumonia in AIDS and other immunosuppressed patients), Toxoplasma gondii, Entamoeba histolytica, Entamoeba invadens, and Giardia lamblia. In certain embodiments, the disclosed compounds are useful for the treatment of parasitic infections in animals and livestock caused by a protozoan parasite selected from Plasmodium hermani, Cryptosporidium sps., Echinococcus granulosus, Eimeria tenella, Sarcocystis neurona, and Neurospora crassa. Other compounds useful as proteasome inhibitors in the treatment of parasitic diseases are described in WO 98/10779, which is herein incorporated by reference in its entirety.

In certain embodiments, the disclosed compounds inhibit proteasome activity irreversibly in a parasite. Such irreversible inhibition has been shown to induce shutdown in enzyme activity without recovery in red blood cells and white blood cells. In certain such embodiments, the long half-life of blood cells may provide prolonged protection with regard to therapy against recurring exposures to parasites. In certain embodiments, the long half-life of blood cells may provide prolonged protection with regard to chemoprophylaxis against future infection, e.g., due to the irreversible binding of the inhibitor and/or long half-life of the blood cells.

It has also been demonstrated that inhibitors that bind to the 20S proteasome stimulate bone formation in bone organ cultures. Furthermore, when such inhibitors have been administered systemically to mice, certain proteasome inhibitors increased bone volume and bone formation rates over 70% (Garrett, I. R. et al., J. Clin. Invest. (2003) 111: 1771-1782), therefore suggesting that the ubiquitin-proteasome machinery regulates osteoblast differentiation and bone formation. Therefore, the disclosed compounds may be useful in the treatment and/or prevention of diseases associated with bone loss, such as osteoporosis.

Bone tissue is an excellent source for factors which have the capacity for stimulating bone cells. Thus, extracts of bovine bone tissue contain not only structural proteins which are responsible for maintaining the structural integrity of bone, but also biologically active bone growth factors which can stimulate bone cells to proliferate. Among these latter factors is a recently described family of proteins called bone morphogenetic proteins (BMPs). All of these growth factors have effects on other types of cells, as well as on bone cells, including Hardy, M. H., et al., Trans Genet (1992) 8:55-61 describes evidence that bone morphogenetic proteins (BMPs), are differentially expressed in hair follicles during development. Harris, S. E., et al., J Bone Miner Res (1994) 9:855-863 describes the effects of TGF-β on expression of BMP-2 and other substances in bone cells. BMP-2 expression in mature follicles also occurs during maturation and after the period of cell proliferation (Hardy, et al. (1992, supra). Thus, compounds of the invention may also be useful for hair follicle growth stimulation.

In certain embodiments, the disclosed compounds are useful in the determination of biodistribution of an inhibitor following administration to a biological sample. These biological samples include, but are not limited to, purified 20S, purified 26S, patient tissue (including blood, aspirates, and biopsies (fresh or frozen), cells (tissue culture), and whole animals (preferably mammals). Monitoring of the biodistribution of the inhibitor may then be performed. In certain embodiments, a fluorescent proteasome inhibitor is administered and the biodistribution of the compound is then determined using fluorescence microscopy or whole animal imaging. Alternatively, the binding of the fluorescent proteasome inhibitor may be determined using fluorescence polarization. In certain embodiments, where the proteasome inhibitor comprises a moiety that selectively binds to an antibody, the technique further comprises administering a secondary antibody that binds to the label of the proteasome inhibitor.

In certain embodiments, the disclosed compounds are useful in a pharmacodynamic assay for identifying and characterizing the binding characteristics of potential proteasome inhibitors. In preferred embodiments, the tagged proteasome inhibitor is useful in a pharmacodynamic assay that quantitates the available 20S proteasome enzymatic active sites before and after administration of a proteasome inhibitor to a mammal.

The assay may be used to determine drug occupancy of the proteasome 20S enzymatic active sites by determining the amount of a known tagged inhibitor that binds to a test biological sample obtained from a mammal treated with a proteasome inhibitor and comparing it to the amount of a known tagged inhibitor that binds to a reference biological sample obtained from a mammal not treated with an inhibitor. A biological sample may be selected from blood (whole blood, PBMC, white blood cells), urine, organ or tissue samples (both intact and dissociated) such as tumor biopsies, skin biopsies, colon biopsies, synovial fluid, bronchial fluid, muscle cells, and bone marrow/blood cell precursors.

The means of detection used to detect the tag is dependent on the nature of the tag used and the nature of the biological sample used, and may include fluorescence polarization, high performance liquid chromatography, antibody capture, gel electrophoresis, differential precipitation, organic extraction, or size exclusion chromatography, fluorescence microscopy, or fluorescence activated cell sorter (FACS) assay.

In another embodiment, the invention provides assays for evaluating proteasome inhibitors. The assays involve separating inhibitor-bound proteasome subunits from unbound subunits and detecting the amount of bound subunits as compared to a control. In an exemplary embodiment, an antibody that binds to a proteasome subunit may be used to detect the amount of inhibitor-bound and/or unbound subunits. Use of the antibodies permits differentiation between inhibitors of the constitutive proteasome, the immunoproteasome and/or other cellular proteases. In various embodiments, one or more antibodies that recognize a constitutive subunit (such as, for example, a β1, β2, or β5 subunit) or an immunoproteasome subunit (such as, for example, a LMP2, MECL 1, or LMP7 subunit) may be used.

The assays may be useful for screening, monitoring, diagnostic and/or dosing purposes. For example, the assays described herein may be used to screen for novel proteasome inhibitors, including inhibitors that may be specific for the constitutive proteasome, the immunoproteasome and/or other cellular proteases. The assays may also be useful for monitoring and/or optimizing a course of therapeutic treatment, including determination of the level of inhibition achieved, monitoring duration of inhibition, and monitoring recovery of proteasome activity over the course of a dosing treatment. For example, in certain embodiments, it may be desirable to completely inhibit the activity of one or more proteases, partially inhibit one or more proteases, and/or temporarily inhibit protease activity to a certain degree. The assays described herein permit monitoring of the level of inhibition achieved and subsequent alteration of the dosing regimen to obtain a desired level of proteasome inhibition by changing the amount and/or frequency of administration of a proteasome inhibitor. In an exemplary embodiment, biological samples are obtained from a patient prior to treatment with a proteasome inhibitor and at one or more specified time intervals after treatment to permit monitoring and/or adjustment of the course of treatment using the assays described herein.

In one embodiment, the assays involve determination of the amount of inhibitor-bound and/or unbound proteasome subunits using a compound having a detectable label that binds to a proteasome inhibitor. Detectable labels that may be used in association with the assays described herein include, for example, a fluorescent moiety, a radioactive isotope-containing moiety, biotin, and a moiety that selectively binds to an antibody. In exemplary embodiments, the assays utilize a tagged proteasome inhibitor such as those described herein, e.g., a compound of any one of formulae I to IV. When using compounds having a fluorescent label, the amount of inhibitor-bound and/or unbound proteasome subunits may be determined by monitoring the amount of fluorescence polarization upon interaction of the compound and the proteasome subunits, e.g., the intensity of the fluorescence signal will be proportional to the amount of bound or unbound subunits. When using compounds having a radioactive isotope-containing moiety, the amount of inhibitor-bound and/or unbound proteasome subunits may be determined by monitoring the amount of radioactive signal retained after binding to the proteasome subunits. For example, the proteasome subunits may be contacted with the compound having a radioactive isotope-containing moiety followed by removal (e.g., by washing, filtration, size separation, etc.) of any compound that did not bind to the proteasome subunits. The amount of radioactive signal remaining in the sample will be directly proportional to the amount of subunits that bound to the compound, e.g., the number of inhibitor-unbound subunits. When using compounds having an affinity tag, e.g., biotin or a moiety that selectively binds to an antibody, the amount of inhibitor-bound and/or unbound subunits may be determined by separating inhibitor-bound and unbound compounds (described below) or by determining the amount of proteasome subunits retained in a sample that contain the compound having the affinity tag. For example, the proteasome subunits may be contacted with the compound having an affinity tag followed by removal (e.g., by washing, filtration, size separation, etc.) of any compound that did not bind to the proteasome subunits. The affinity tag may then be detected, for example, using streptavidin or an antibody having a detectable label. The amount of the affinity tag labeled compound remaining in the sample will be directly proportional to the amount of subunits that bound to the compound, e.g., the number of inhibitor-unbound subunits.

In various embodiments, the assays may be carried out on samples to determine the activity of a proteasome. In an exemplary embodiment, the assays may be used to determine the drug occupancy of the proteasome 20S chymotryptic active site. For example, a biological sample may be contacted with a known or potential proteasome inhibitor. The biological sample is then contacted with a compound having a detectable label that binds to a proteasome inhibitor. The compound will only bind to the active sites that are not occupied by the proteasome inhibitor. The amount of proteasome subunits that bound to the compound will be directly proportional to the amount of free active sites, e.g., the number of proteasome subunits that did not bind to the known or potential proteasome inhibitor;

In one embodiment, the assays involve separation of inhibitor-bound proteasome subunits from unbound subunits by direct isolation of the inhibitor-bound subunits. For example, inhibitor-bound subunits may be isolated using a reagent that specifically binds to the inhibitor, such as, for example, an inhibitor-specific antibody. Methods for producing antibodies are well known in the art. In certain embodiments, the inhibitor may be tagged with a detectable label that permits isolation of the inhibitor-bound subunits by affinity interaction with the label. Examples of detectable labels include, for example, a fluorescent moiety, a radioactive isotope-containing moiety, biotin, and a moiety that selectively binds to an antibody. In an exemplary embodiment, the proteasome inhibitor is a compound as described herein (e.g., a compound of any one of formulae I to IV) that may be used to directly isolate inhibitor-bound proteasome subunits through interaction with a detectable label.

In another embodiment, a tagged inhibitor that binds to a proteasome subunit may be used to facilitate separation of inhibitor-bound and unbound proteasome subunits. For example, a sample may be treated with a proteasome inhibitor that will occupy all or a portion of the active sites of the proteasome subunits. The proteasome inhibitor may be untagged or may be labeled with a tag different from the tag included in the tagged inhibitor. The sample may then be treated with a tagged inhibitor that binds to the active site of the proteasome subunits. The tagged inhibitor will bind to the free active sites, e.g., the sites that are not occupied by the proteasome inhibitor. An affinity reagent that binds to the tagged inhibitor may then be used to separate the inhibitor-bound proteasome subunits from the unbound subunits, e.g., the unbound subunits will bind to the tagged inhibitor while the inhibitor-bound subunits will not. In an exemplary embodiment, the tagged inhibitor is a compound disclosed herein (e.g., a compound of any one of formulae I to IV).

In certain embodiments, the activity of a proteasome inhibitor may be evaluated by determining the amount of inhibitor-bound and/or unbound subunits, for example, a ratio of inhibitor-bound and unbound subunits. In other embodiments, the amount of inhibitor-bound and/or unbound proteasome subunits in a given sample may be compared to the amount of bound and/or unbound proteasome subunits in a control sample. For example, a control may be a sample that has not been treated with a proteasome inhibitor, a sample prior to treatment with an inhibitor, or a sample that has been treated with a control reagent (e.g., an inhibitor known to have a given level of activity, etc.). In other embodiments, a control may be one or more data points that have been previously determined and stored in an accessible format, including in a database such as a computer accessible database. For example, a control may be a data set of inhibitor-bound and/or unbound levels for a given proteasome inhibitor, such as, for example a data set from a large patient population. In one embodiment, a control may be a standard curve of bound and/or unbound levels after treatment with varying amounts of a given proteasome inhibitor. In an exemplary embodiment, a control may be a biological sample from a subject prior to administration of a proteasome inhibitor. In certain embodiments, the amount of inhibitor-bound and/or unbound proteasome subunits may be determined quantitatively and/or qualitatively.

The activity of any type of proteasome inhibitor can be evaluated using the assays described herein. Non-limiting examples of proteasome inhibitors include, for example, peptide aldehydes (see, e.g., PCT Publication Nos. WO 95/24914, WO 91/13904; Iqbal et al. J. Med. Chem. 38:2276-2277 (1995)), vinyl sulfones (see, e.g., Bogyo et al., Proc. Natl. Acad. Sci. 94:6629 (1997)), a'β'-epoxyketones (see, e.g., Spaltenstein et al. Tetrahedron Lett. 37:1343 (1996); U.S. Patent No. 6,831,099); peptide boronic acids (see, e.g., PCT Publication Nos. WO 96/13266 and WO 91/13904), and lactacystin and lactacystin analogs (see, e.g., Fenteany et al. Proc. Natl. Acad. Sci. USA 94:3358 (1994); PCT Publication No. WO 96/32105), each of which is hereby incorporated by reference in its entirety. In one embodiment, the proteasome inhibitors that may be evaluated using the methods disclosed herein include both irreversible and reversible covalently bound proteasome inhibitors or inhibitors having a higher binding affinity (e.g., a lower K_{d}) for a proteasome subunit than the binding affinity for a tagged inhibitor disclosed herein.

Variability in sample preparation can be controlled by introducing a normalization process into the assay format. For example, proteasome activity (as determined by the amount of inhibitor-bound and/or unbound proteasome subunits) in a sample may be normalized relative to the protein content in the sample. Total protein content in the sample can be determined using standard procedures, including, without limitation, a Bradford assay or the Lowry method.

In other embodiments, the assays described herein may be used for diagnostic purposes or prognostic purposes. For example, levels of proteasome activity (as determined by the amount of inhibitor-bound and/or unbound proteasome subunits) may distinguish between different disease states that give rise to similar symptoms. Similarly, mammals with a particular disease may be divided into subpopulations according to baseline proteasome activity level. In an exemplary embodiment, the methods described herein may be used to correlate the level of constitutive proteasome and/or immunoproteasome activity with a given disease state or risk level for a given disease.

Finally, the disclosed compounds are also useful as diagnostic agents (e.g., in diagnostic kits or for use in clinical laboratories) for screening for proteins (e.g., enzymes, transcription factors) processed by Ntn hydrolases, including the proteasome. The disclosed compounds are also useful as research reagents for specifically binding the X/MB1 subunit or a-chain and inhibiting the proteolytic activities associated with it. For example, the activity of (and specific inhibitors of) other subunits of the proteasome can be determined.

Most cellular proteins are subject to proteolytic processing during maturation or activation. Enzyme inhibitors disclosed herein can be used to determine whether a cellular, developmental, or physiological process or output is regulated by the proteolytic activity of a particular Ntn hydrolase. One such method includes obtaining an organism, an intact cell preparation, or a cell extract; exposing the organism, cell preparation, or cell extract to a compound disclosed herein; exposing the compound-exposed organism, cell preparation, or cell extract to a signal, and monitoring the process or output. The high selectivity of the compounds disclosed herein permits rapid and accurate elimination or implication of the Ntn (for example, the 20S proteasome) in a given cellular, developmental, or physiological process.

### Administration

Compounds prepared as described herein can be administered in various forms, depending on the disorder to be treated and the age, condition, and body weight of the patient, as is well known in the art. For example, where the compounds are to be administered orally, they may be formulated as tablets, capsules, granules, powders, or syrups; or for parenteral administration, they may be formulated as injections (intravenous, intramuscular, or subcutaneous), drop infusion preparations, or suppositories. For application by the ophthalmic mucous membrane route, they may be formulated as eye drops or eye ointments. These formulations can be prepared by conventional means, and, if desired, the active ingredient may be mixed with any conventional additive or excipient, such as a binder, a disintegrating agent, a lubricant, a corrigent, a solubilizing agent, a suspension aid, an emulsifying agent, a coating agent, a cyclodextrin, and/or a buffer. Although the dosage will vary depending on the symptoms, age and body weight of the patient, the nature and severity of the disorder to be treated or prevented, the route of administration and the form of the drug, in general, a daily dosage of from 0.01 to 2000 mg of the compound is recommended for an adult human patient, and this may be administered in a single dose or in divided doses. The amount of active ingredient which can be combined with a carrier material to produce a single dosage form will generally be that amount of the compound which produces a therapeutic effect.

The precise time of administration and/or amount of the inhibitor that will yield the most effective results in terms of efficacy of treatment in a given patient will depend upon the activity, pharmacokinetics, and bioavailability of a particular compound, physiological condition of the patient (including age, sex; disease type and stage, general physical condition, responsiveness to a given dosage, and type of medication), route of administration, etc. However, the above guidelines can be used as the basis for fine-tuning the treatment, e.g., determining the optimum time and/or amount of administration, which will require no more than routine experimentation consisting of monitoring the subject and adjusting the dosage and/or timing.

The phrase "pharmaceutically acceptable" is employed herein to refer to those ligands, materials, compositions, and/or dosage forms which are, within the scope of sound medical judgment, suitable for use in contact with the tissues of human beings and animals without excessive toxicity, irritation, allergic response, or other problem or complication, commensurate with a reasonable benefit/risk ratio.

The phrase "pharmaceutically acceptable carrier" as used herein means a pharmaceutically acceptable material, composition or vehicle, such as a liquid or solid filler, diluent, excipient, solvent or encapsulating material, involved in carrying or transporting the subject chemical from one organ or portion of the body, to another organ or portion of the body. Each carrier must be "acceptable" in the sense of being compatible with the other ingredients of the formulation and not injurious to the patient. Some examples of materials which can serve as pharmaceutically acceptable carriers include: (1) sugars, such as lactose, glucose, and sucrose; (2) starches, such as corn starch and potato starch; (3) cellulose, and its derivatives, such as sodium carboxymethyl cellulose, ethyl cellulose, and cellulose acetate; (4) powdered tragacanth; (5) malt; (6) gelatin; (7) talc; (8) excipients, such as cocoa butter and suppository waxes; (9) oils, such as peanut oil, cottonseed oil, safflower oil, sesame oil, olive oil, corn oil, and soybean oil; (10) glycols, such as propylene glycol; (11) polyols, such as glycerin, sorbitol, mannitol, and polyethylene glycol; (12) esters, such as ethyl oleate and ethyl laurate; (13) agar; (14) buffering agents, such as magnesium hydroxide and aluminum hydroxide; (15) alginic acid; (16) pyrogen-free water; (17) isotonic saline; (18) Ringer's solution; (19) ethyl alcohol; (20) phosphate buffer solutions; and (21) other non-toxic compatible substances employed in pharmaceutical formulations. In certain embodiments, pharmaceutical compositions of the present invention are non-pyrogenic, i.e., do not induce significant temperature elevations when administered to a patient.

The term "pharmaceutically acceptable salt" refers to the relatively non-toxic, inorganic and organic acid addition salts of the inhibitor(s). These salts can be prepared *in situ* during the final isolation and purification of the inhibitor(s), or by separately reacting a purified inhibitor(s) in its free base form with a suitable organic or inorganic acid, and isolating the salt thus formed. Representative salts include the hydrobromide, hydrochloride, sulfate, bisulfate, phosphate, nitrate, acetate, valerate, oleate, palmitate, stearate, laurate, benzoate, lactate, phosphate, tosylate, citrate, maleate, fumarate, succinate, tartrate, naphthylate, mesylate, glucoheptonate, lactobionate, laurylsulphonate salts, and amino acid salts, and the like. (See, for example, Berge et al. (1977) "Pharmaceutical Salts", J. Pharm. Sci. 66: 1-19.)

In other cases, the inhibitors useful in the methods of the present invention may contain one or more acidic functional groups and, thus, are capable of forming pharmaceutically acceptable salts with pharmaceutically acceptable bases. The term "pharmaceutically acceptable salts" in these instances refers to the relatively non-toxic inorganic and organic base addition salts of an inhibitor(s). These salts can likewise be prepared in situ during the final isolation and purification of the inhibitor(s), or by separately reacting the purified inhibitor(s) in its free acid form with a suitable base, such as the hydroxide, carbonate, or bicarbonate of a pharmaceutically acceptable metal cation, with ammonia, or with a pharmaceutically acceptable organic primary, secondary, or tertiary amine. Representative alkali or alkaline earth salts include the lithium, sodium, potassium, calcium, magnesium, and aluminum salts, and the like. Representative organic amines useful for the formation of base addition salts include ethylamine, diethylamine, ethylenediamine, ethanolamine, diethanolamine, piperazine, and the like (see, for example, Berge et al., *supra*)*.*

Wetting agents, emulsifiers, and lubricants, such as sodium lauryl sulfate and magnesium stearate, as well as coloring agents, release agents, coating agents, sweetening, flavoring, and perfuming agents, preservatives and antioxidants can also be present in the compositions.

Examples of pharmaceutically acceptable antioxidants include: (1) water soluble antioxidants, such as ascorbic acid, cysteine hydrochloride, sodium bisulfate, sodium metabisulfite, sodium sulfite, and the like; (2) oil-soluble antioxidants, such as ascorbyl palmitate, butylated hydroxyanisole (BHA), butylated hydroxytoluene (BHT), lecithin, propyl gallate, alpha-tocopherol, and the like; and (3) metal chelating agents, such as citric acid, ethylenediamine tetraacetic acid (EDTA), sorbitol, tartaric acid, phosphoric acid, and the like.

Formulations suitable for oral administration may be in the form of capsules, cachets, pills, tablets, lozenges (using a flavored basis, usually sucrose and acacia or tragacanth), powders, granules, or as a solution or a suspension in an aqueous or nonaqueous liquid, or as an oil-in-water or water-in-oil liquid emulsion, or as an elixir or syrup, or as pastilles (using an inert matrix, such as gelatin and glycerin, or sucrose and acacia) and/or as mouthwashes, and the like, each containing a predetermined amount of an inhibitor(s) as an active ingredient. A compound may also be administered as a bolus, electuary or paste.

In solid dosage forms for oral administration (capsules, tablets, pills, dragees, powders, granules, and the like), the active ingredient is mixed with one or more pharmaceutically acceptable carriers, such as sodium citrate or dicalcium phosphate, and/or any of the following: (1) fillers or extenders, such as starches, lactose, sucrose, glucose, mannitol, and/or silicic acid; (2) binders, such as, for example, carboxymethylcellulose, alginates, gelatin, polyvinyl pyrrolidone, sucrose, and/or acacia; (3) humectants, such as glycerol; (4) disintegrating agents, such as agar-agar, calcium carbonate, potato or tapioca starch, alginic acid, certain silicates, and sodium carbonate; (5) solution retarding agents, such as paraffin; (6) absorption accelerators, such as quaternary ammonium compounds; (7) wetting agents, such as, for example, acetyl alcohol and glycerol monostearate; (8) absorbents, such as kaolin and bentonite clay; (9) lubricants, such a talc, calcium stearate, magnesium stearate, solid polyethylene glycols, sodium lauryl sulfate, and mixtures thereof; and (10) coloring agents. In the case of capsules, tablets, and pills, the pharmaceutical compositions may also comprise buffering agents. Solid compositions of a similar type may also be employed as fillers in soft and hard-filled gelatin capsules using such excipients as lactose or milk sugars, as well as high molecular weight polyethylene glycols, and the like.

A tablet may be made by compression or molding, optionally with one or more accessory ingredients. Compressed tablets may be prepared using binder (for example, gelatin or hydroxypropylmethyl cellulose), lubricant, inert diluent, preservative, disintegrant (for example, sodium starch glycolate or cross-linked sodium carboxymethyl cellulose), surface-active or dispersing agent. Molded tablets may be made by molding in a suitable machine a mixture of the powdered peptide or peptidomimetic moistened with an inert liquid diluent.

Tablets, and other solid dosage forms, such as dragees, capsules, pills, and granules, may optionally be scored or prepared with coatings and shells, such as enteric coatings and other coatings well known in the pharmaceutical-formulating art. They may also be formulated so as to provide slow or controlled release of the active ingredient therein using, for example, hydroxypropylmethyl cellulose in varying proportions to provide the desired release profile, other polymer matrices, liposomes, and/or microspheres. They may be sterilized by, for example, filtration through a bacteria-retaining filter, or by incorporating sterilizing agents in the form of sterile solid compositions which can be dissolved in sterile water, or some other sterile injectable medium immediately before use. These compositions may also optionally contain opacifying agents and may be of a composition that they release the active ingredient(s) only, or preferentially, in a certain portion of the gastrointestinal tract, optionally, in a delayed manner. Examples of embedding compositions which can be used include polymeric substances and waxes. The active ingredient can also be in micro-encapsulated form, if appropriate, with one or more of the above-described excipients.

Liquid dosage forms for oral administration include pharmaceutically acceptable emulsions, microemulsions, solutions, suspensions, syrups, and elixirs. In addition to the active ingredient, the liquid dosage forms may contain inert diluents commonly used in the art, such as, for example, water or other solvents, solubilizing agents, and emulsifiers such as ethyl alcohol, isopropyl alcohol, ethyl carbonate, ethyl acetate, benzyl alcohol, benzyl benzoate, propylene glycol, 1,3-butylene glycol, oils (in particular, cottonseed, groundnut, corn, germ, olive, castor, and sesame oils), glycerol, tetrahydrofuryl alcohol, polyethylene glycols, and fatty acid esters of sorbitan, and mixtures thereof.

Besides inert diluents, the oral compositions can also include adjuvants such as wetting agents, emulsifying and suspending agents, sweetening, flavoring, coloring, perfuming, and preservative agents.

Suspensions, in addition to the active inhibitor(s) may contain suspending agents as, for example, ethoxylated isostearyl alcohols, polyoxyethylene sorbitol and sorbitan esters, microcrystalline cellulose, aluminum metahydroxide, bentonite, agar-agar and tragacanth, and mixtures thereof.

Formulations for rectal or vaginal administration may be presented as a suppository, which may be prepared by mixing one or more inhibitor(s) with one or more suitable nonirritating excipients or carriers comprising, for example, cocoa butter, polyethylene glycol, a suppository wax or a salicylate, which is solid at room temperature, but liquid at body temperature and, therefore, will melt in the rectum or vaginal cavity and release the active agent.

Formulations which are suitable for vaginal administration also include pessaries, tampons, creams, gels, pastes, foams, or spray formulations containing such carriers as are known in the art to be appropriate.

Dosage forms for the topical or transdermal administration of an inhibitor(s) include powders, sprays, ointments, pastes, creams, lotions, gels, solutions, patches, and inhalants. The active component may be mixed under sterile conditions with a pharmaceutically acceptable carrier, and with any preservatives, buffers, or propellants which may be required.

The ointments, pastes, creams, and gels may contain, in addition to inhibitor(s), excipients, such as animal and vegetable fats, oils, waxes, paraffins, starch, tragacanth, cellulose derivatives, polyethylene glycols, silicones, bentonites, silicic acid, talc, and zinc oxide, or mixtures thereof.

Powders and sprays can contain, in addition to an inhibitor(s), excipients such as lactose, talc, silicic acid, aluminum hydroxide, calcium silicates, and polyamide powder, or mixtures of these substances. Sprays can additionally contain customary propellants, such as chlorofluorohydrocarbons and volatile unsubstituted hydrocarbons, such as butane and propane.

The inhibitor(s) can be alternatively administered by aerosol. This is accomplished by preparing an aqueous aerosol, liposomal preparation, or solid particles containing the compound. A nonaqueous (e.g., fluorocarbon propellant) suspension could be used. Sonic nebulizers are preferred because they minimize exposing the agent to shear, which can result in degradation of the compound.

Ordinarily, an aqueous aerosol is made by formulating an aqueous solution or suspension of the agent together with conventional pharmaceutically acceptable carriers and stabilizers. The carriers and stabilizers vary with the requirements of the particular compound, but typically include nonionic surfactants (Tweens, Pluronics, sorbitan esters, lecithin, Cremophors), pharmaceutically acceptable co-solvents such as polyethylene glycol, innocuous proteins like serum albumin, oleic acid, amino acids such as glycine, buffers, salts, sugars, or sugar alcohols. Aerosols generally are prepared from isotonic solutions.

Transdermal patches have the added advantage of providing controlled delivery of an inhibitor(s) to the body. Such dosage forms can be made by dissolving or dispersing the agent in the proper medium. Absorption enhancers can also be used to increase the flux of the inhibitor(s) across the skin. The rate of such flux can be controlled by either providing a rate controlling membrane or dispersing the peptidomimetic in a polymer matrix or gel.

Pharmaceutical compositions of this invention suitable for parenteral administration comprise one or more inhibitors(s) in combination with one or more pharmaceutically acceptable sterile aqueous or nonaqueous solutions, dispersions, suspensions or emulsions, or sterile powders which may be reconstituted into sterile injectable solutions or dispersions just prior to use, which may contain antioxidants, buffers, bacteriostats, solutes which render the formulation isotonic with the blood of the intended recipient or suspending or thickening agents.

Examples of suitable aqueous and nonaqueous carriers which may be employed in the pharmaceutical compositions of the invention include water, ethanol, polyols (such as glycerol, propylene glycol, polyethylene glycol, and the like), and suitable mixtures thereof, vegetable oils, such as olive oil, and injectable organic esters, such as ethyl oleate. Proper fluidity can be maintained, for example, by the use of coating materials, such as lecithin, by the maintenance of the required particle size in the case of dispersions, and by the use of surfactants.

These compositions may also contain adjuvants such as preservatives, wetting agents, emulsifying agents, and dispersing agents. Prevention of the action of microorganisms may be ensured by the inclusion of various antibacterial and antifungal agents, for example, paraben, chlorobutanol, phenol sorbic acid, and the like. It may also be desirable to include tonicity-adjusting agents, such as sugars, sodium chloride, and the like into the compositions. In addition, prolonged absorption of the injectable pharmaceutical form may be brought about by the inclusion of agents which delay absorption such as aluminum monostearate and gelatin.

In some cases, in order to prolong the effect of a drug, it is desirable to slow the absorption of the drug from subcutaneous or intramuscular injection. This may be accomplished by the use of a liquid suspension of crystalline or amorphous material having poor water solubility. The rate of absorption of the drug then depends upon its rate of dissolution which, in turn, may depend upon crystal size and crystalline form. Alternatively, delayed absorption of a parenterally administered drug form is accomplished by dissolving or suspending the drug in an oil vehicle.

Injectable depot forms are made by forming microencapsule matrices of inhibitor(s) in biodegradable polymers such as polylactide-polyglycolide. Depending on the ratio of drug to polymer, and the nature of the particular polymer employed, the rate of drug release can be controlled. Examples of other biodegradable polymers include poly(orthoesters) and poly(anhydrides). Depot injectable formulations are also prepared by entrapping the drug in liposomes or microemulsions which are compatible with body tissue.

When the inhibitors(s) of the present invention are administered as pharmaceuticals to humans and animals, they can be given per se or as a pharmaceutical composition containing, for example, 0.1 to 99.5% (more preferably, 0.5 to 90%) of active ingredient in combination with a pharmaceutically acceptable carrier.

The preparations of agents may be given orally, parenterally, topically, or rectally. They are, of course, given by forms suitable for each administration route. For example, they are administered in tablets or capsule form, by injection, inhalation, eye lotion, ointment, suppository, infusion; topically by lotion or ointment; and rectally by suppositories. Oral administration is preferred.

The phrases "parenteral administration" and "administered parenterally" as used herein means modes of administration other than enteral and topical administration, usually by injection, and includes, without limitation, intravenous, intramuscular, intraarterial, intrathecal, intracapsular, intraorbital, intracardiac, intradermal, intraperitoneal, transtracheal, subcutaneous, subcuticular, intraarticular, subcapsular, subarachnoid, intraspinal and intrasternal injection, and infusion.

The phrases "systemic administration," "administered systemically," "peripheral administration" and "administered peripherally" as used herein mean the administration of a ligand, drug, or other material other than directly into the central nervous system, such that it enters the patient's system and thus, is subject to metabolism and other like processes, for example, subcutaneous administration.

These inhibitors(s) may be administered to humans and other animals for therapy by any suitable route of administration, including orally, nasally, as by, for example, a spray, rectally, intravaginally, parenterally, intracisternally, and topically, as by powders, ointments or drops, including buccally and sublingually.

Regardless of the route of administration selected, the inhibitor(s), which may be used in a suitable hydrated form, and/or the pharmaceutical compositions of the present invention, are formulated into pharmaceutically acceptable dosage forms by conventional methods known to those of skill in the art.

Actual dosage levels of the active ingredients in the pharmaceutical compositions of this invention may be varied so as to obtain an amount of the active ingredient which is effective to achieve the desired therapeutic response for a particular patient, composition, and mode of administration, without being toxic to the patient.

The concentration of a disclosed compound in a pharmaceutically acceptable mixture will vary depending on several factors, including the dosage of the compound to be administered, the pharmacokinetic characteristics of the compound(s) employed, and the route of administration. In general, the compounds of this invention may be provided in an aqueous solution containing about 0.1-10% w/v of compound for parenteral administration. Typical dose ranges are from about 0.01 to about 50 mg/kg of body weight per day, given in 1-4 divided doses. Each divided dose may contain the same or different compounds of the invention. The dosage will be an effective amount depending on several factors including the overall health of a patient, and the formulation and route of administration of the selected compound(s).

Another aspect of the invention provides a conjoint therapy wherein one or more other therapeutic agents are administered with the protease inhibitor. Such conjoint treatment may be achieved by way of the simultaneous, sequential, or separate dosing of the individual components of the treatment.

In certain embodiments, a compound of the invention is conjointly administered with one or more other proteasome inhibitor(s).

In certain embodiments, a compound of the invention is conjointly administered with a chemotherapeutic. Suitable chemotherapeutics may include, natural products such as vinca alkaloids (i.e., vinblastine, vincristine, and vinorelbine), paclitaxel, epidipodophyllotoxins (i.e., etoposide, teniposide), antibiotics (dactinomycin (actinomycin D) daunorubicin, doxorubicin and idarubicin), anthracyclines, mitoxantrone, bleomycins, plicamycin (mithramycin) and mitomycin, enzymes (L-asparaginase which systemically metabolizes L-asparagine and deprives cells which do not have the capacity to synthesize their own asparagine); antiplatelet agents; antiproliferative/antimitotic alkylating agents such as nitrogen mustards (mechlorethamine, cyclophosphamide and analogs, melphalan, chlorambucil), ethylenimines and methylmelamines (hexamethylmelamine and thiotepa), alkyl sulfonates (busulfan), nitrosoureas (carmustine (BCNU) and analogs, streptozocin), trazenes - dacarbazinine (DTIC); antiproliferative/antimitotic antimetabolites such as folic acid analogs (methotrexate), pyrimidine analogs (fluorouracil, floxuridine, and cytarabine), purine analogs and related inhibitors (mercaptopurine, thioguanine, pentostatin and 2-chlorodeoxyadenosine); aromatase inhibitors (anastrozole, exemestane, and letrozole); and platinum coordination complexes (cisplatin, carboplatin), procarbazine, hydroxyurea, mitotane, aminoglutethimide; histone deacetylase (HDAC) inhibitors (trichostatin, sodium butyrate, apicidan, suberoyl anilide hydroamic acid); hormones (i.e. estrogen) and hormone agonists such as leutinizing hormone releasing hormone (LHRH) agonists (goserelin, leuprolide and triptorelin). Other chemotherapeutic agents may include mechlorethamine, camptothecin, ifosfamide, tamoxifen, raloxifene, gemcitabine, navelbine, or any analog or derivative variant of the foregoing.

In certain embodiments, a compound of the invention is conjointly administered with a cytokine. Cytokines include, but are not limited to, Interferon-γ, -α, and -β, Interleukins 1-8, 10 and 12, Granulocyte Monocyte Colony-Stimulating factor (GM-CSF), TNF-α and -β, and TGF-β.

In certain embodiments, a compound of the invention is conjointly administered with a steroid. Suitable steroids may include, but are not limited to, 21-acetoxypregnenolone, alclometasone, algestone, amcinonide, beclomethasone, betamethasone, budesonide, chloroprednisone, clobetasol, clocortolone, cloprednol, corticosterone, cortisone, cortivazol, deflazacort, desonide, desoximetasone, dexamethasone, diflorasone, diflucortolone, difuprednate, enoxolone, fluazacort, flucloronide, flumethasone, flunisolide, fluocinolone acetonide, fluocinonide, fluocortin butyl, fluocortolone, fluorometholone, fluperolone acetate, fluprednidene acetate, fluprednisolone, flurandrenolide, fluticasone propionate, formocortal, halcinonide, halobetasol propionate, halometasone, hydrocortisone, loteprednol etabonate, mazipredone, medrysone, meprednisone, methylprednisolone, mometasone furoate, paramethasone, prednicarbate, prednisolone, prednisolone 25-diethylaminoacetate, prednisolone sodium phosphate, prednisone, prednival, prednylidene, rimexolone, tixocortol, triamcinolone, triamcinolone acetonide, triamcinolone benetonide, triamcinolone hexacetonide, and salts and/or derivatives thereof.

In certain embodiments, a compound of the invention is conjointly administered with an immunotherapeutic agent. Suitable immunotherapeutic agents may include, but are not limited to, MDR modulators (verapamil, valspodar, biricodar, tariquidar, laniquidar), cyclosporine, thalidomide, and monoclonal antibodies. The monoclonal antibodies can be either naked or conjugated such as rituximab, tositumomab, alemtuzumab, epratuzumab, ibritumomab tiuxetan, gemtuzumab ozogamicin, bevacizumab, cetuximab, erlotinib and trastuzumab.

### Exemplification

### Example 1: Synthesis of Tagged Inhibitors 1 and 2

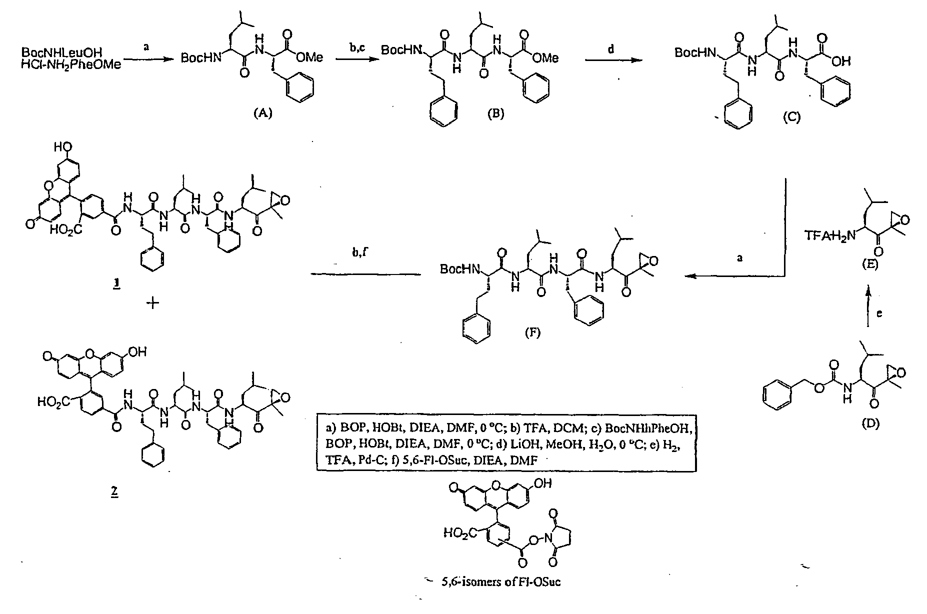

### Synthesis of (A)

To a solution of NBoc Leucine (11.56 g, 50.0 mmol, 1.0 eq.) and phenylalanine methyl ester (10.78 g, 50.0 mmol, 1.0 eq.) in 500 mL of DMF is added HOBT (10.81 g, 80.0 mmol, 1.6 eq.) and DIEA (25.85 g, 35 mL, 200.0 mmol, 4.0 eq.). The mixture is cooled to 0 °C in an ice-water bath and BOP (35.38 g, 80.0 mmol, 1.6 eq.) is added in several portions over five minutes. The reaction is placed under an atmosphere of argon and stirred overnight. The reaction is diluted with brine (1000 mL) and extracted with EtOAc (5 x 200 mL). The organic layers are combined and washed with water (10 x 100 mL), brine (2 x 150 mL) and dried over MgSO₄. The MgSO₄ is removed by filtration and the volatiles are removed under reduced pressure to give (A).

### Synthesis of (B)

To a 50 mL 0 °C cooled solution of 80% TFA/DCM is added compound A (18.0 g, 45.86 mmol, 1.0 eq.). The solution is stirred and allowed to warm to room temperature slowly. The reaction is followed by TLC until complete. The volatiles are then removed under reduced pressure and the resulting material is redissolved in 100 mL of DCM and evaporated. This redissolve and evaporation process is repeated three times. To the resultant material is added BocNHh-Phe (12.81 g, 45.86 mmol, 1.0 eq.), 500 mL of DMF, HOBT (9.91 g, 73.37 mmol, 1.6 eq.) and DIEA (23.70 g, 32.0 mL, 183.44 mmol, 4.0 eq.). The mixture is cooled to 0 °C in an ice-water bath and BOP (32.45 g, 73.37 mmol, 1.6 eq.) is added in several portions over five minutes. The reaction is placed under argon and allowed to warm to room temperature. The reaction is followed by TLC until complete. The reaction is then diluted with 1500 mL of H₂O and extracted with DCM (5 x 300 mL). The organic layers are combined and washed with H₂O (6 x 300 mL), brine (1 x 300 mL), and dried over MgSO₄. The MgSO₄ is removed by filtration and the volatiles removed under reduced pressure to give (B).

### Synthesis of (C)

Compound (B) (232 mg, 0.42 mmol) is dissolved in 3:1 MeOH:H:₂O (20 mL) and then cooled to 0 °C. A solution of 4.2 M LiOH (1 mL, 4.2 mmol, 10 eq) is added followed by stirring at 0 °C over night. The reaction is quenched via addition of saturated NH₄Cl. The resulting mixture is then poured into ice-cold H₂O (30 mL) followed by slow addition of 1N HCl to adjust the pH to ∼3. This solution is then washed with DCM (6x30 mL). The combined organic washes are then dried with Na₂SO₄, filtered, and evaporated under reduced pressure to afford acid (C).

### Synthesis of (F)

To a flask containing (D) (25 mg, 0.082 mmol, 1.0 eq.) is added 5% Pd/C (12 mg, ∼0.25 x weight of (D) U.S. Patent Application Serial No. 11/131688) and the contents placed under argon. To this flask is added TFA (2 mL). The flask is flushed with H₂ and finally placed under an atmosphere of H₂ with a double walled balloon (∼1 atm). The reaction is followed by TLC and HPLC until deemed complete. The flask is then flushed with argon and the contents diluted with 15 mL of DCM and filtered through a pad of Celite. The volatiles are removed under reduced pressure and the oil dissolved in DCM. The volatiles are once again removed under reduced pressure. This process is completed a total of 3 times. The crude amine salt (E) is then placed under high vacuum for 1.5 hours. To (E) is added (C) (44 mg, 0.082 mmol, 1.0 eq.) and DMF (2 mL). The contents are cooled in a 0 °C ice-water bath and DIEA (0.328 mg, 0.057 mL, 3.80 mmol, 4.0 eq.) is added. To the mixture is then added HOBT (18 mg, 0.131 mmol, 1.6 eq.) followed by BOP (58 mg, 0.131 mmol, 1.6 eq). The mixture is then placed under an atmosphere of argon and stirred at 5 °C. Once the reaction is deemed complete using RP-HPLC analysis, the reaction mixture is poured into 50 mL of ice-water, and then extracted with EtOAc (3 x 30 mL). The combined organics are then washed with H₂O (30 mL), NaHCO₃ (30 mL), and brine (30 mL). After drying with MgSO₄, filtering, and evaporating to dryness, the desired compound (F) is obtained.

### Synthesis of Target Inhibitors 1 and 2

To (F) (97 mg, 0.14 mmol, 1.0 eq.) at room temperature is added 5 mL of 80% TFA/DCM. The reaction is followed by TLC until complete. The volatiles are then removed under reduced pressure and the resulting material is redissolved in 100 mL of DCM and evaporated. This redissolve and evaporation process is repeated three times. To the resultant material is added 5,6-carboxyfluorescein succinimidyl ester (isomeric mixture) (68 mg, 0.143 mmol) and DMF (5 mL) followed by addition of DIEA (0.2 mL). The resultant solution is stirred until the reaction is complete. The mixture is then poured into ice-cold H₂O (100 mL) followed by addition of 6M HCl to adjust the pH to 3. After stirring briefly, the precipitate is filtered, rinsed with ice-cold H₂O (50 mL) and then dried under high vacuum to afford a mixture of Inhibitor 1 and Inhibitor 2. RP-HPLC purification provides the single isomers.

### Example 2: Activity of Fluorescent Proteasome Inhibitor

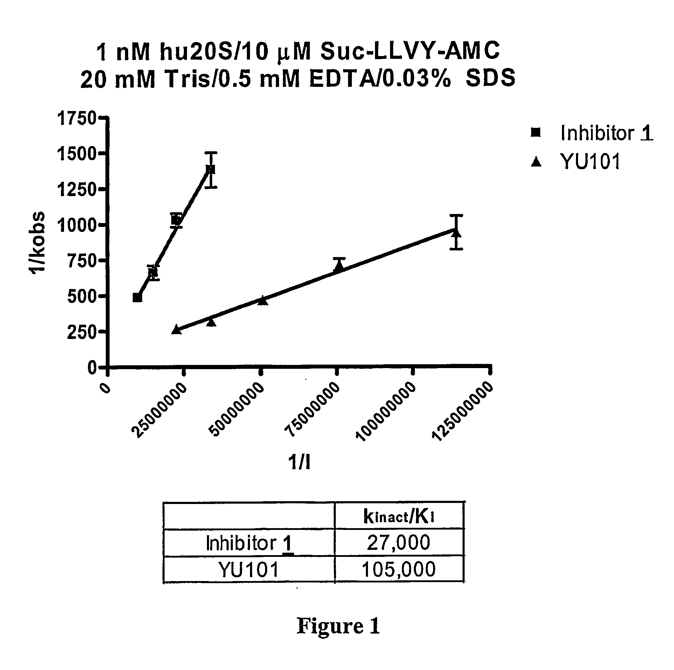

The ability of Inhibitor 1 to inhibit the proteolytic activity of the human 20S proteasome (hu20S) both *in vitro* and in cells was tested using the fluorescent substrate for the chymotryptic-like activity of the proteasome, Suc-LLVY-AMC as shown in Figure 1 and Table 1. Fluorescence was measured using the excitation wavelength of 340 nm and an emission wavelength of 465 nm. When the concentration of Suc-LLVY-AMC is 10 µM, and the 1 nM hu20S is used, the k_{inact}/Kᵢ value for Inhibitor 1 is 27,000 M⁻¹sec⁻¹, which is only 4X lower than the non-labeled analog. The assay was carried out in 20 mM Tris, pH 8.0, 0.5 mM EDTA, 0.03% SDS buffer with 5% DMSO.

**Table 1**

| | | | | **Cell Chymotryptic Activity** | | **Cell Viability** | |
|---|---|---|---|---|---|---|---|
| | **Human 20S Chymotryptic Activity** | | | **8226 I hr** | **HT29 4 hr** | **8226 24 hr** | **HT29 72 hr** |
| **Structure** | **k_{inact.}/K_{¡} (M⁻¹sec⁻¹)** | **R_{obs}/[I] (M⁻¹sec⁻¹)** | **V_{f}IC50 (nM)** | **IC50 (nM)** | **IC50 (nM)** | **IC50 (nM)** | **IC50 (nM)** |
| 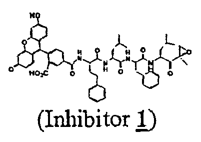 | **27,000** | **23,000** | **9.4** | **340** | | **191** | |
| 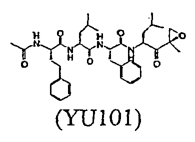 | **105,000** | **93,500** | **2** | **7.3** | **3.3** | **5** | **6.8** |

### Example 3: Measuring Binding to the Proteasome via Fluorescence Polarization

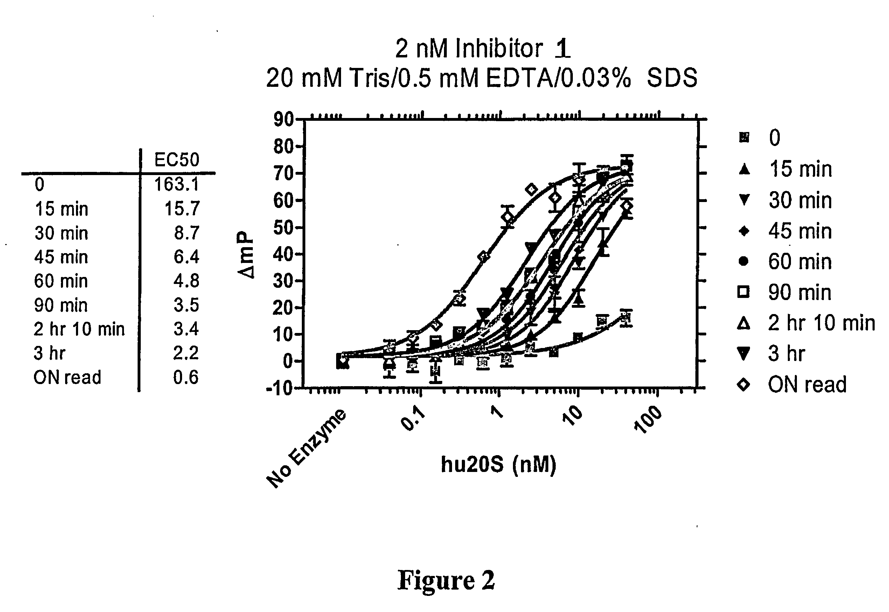

2 nM of Inhibitor 1 was incubated with a serial dilution of purified human 20S proteasome (from 400 pM to 40 nM) in a 20 mM Tris, pH 8.0, 0.5 mM EDTA, 0.03% SDS buffer and was allowed to bind to the proteasome during an incubation at room temperature as shown in Figure 2. Fluorescence polarization was measured by exciting the samples at 485 nm and detecting the emitted fluorescence at 535 nm in a microwell plate in a fluorimeter capable of measuring fluorescence polarization (Tecan GENios Pro). Readings were made at timed intervals and demonstrated an increase in the amount of bound Inhibitor 1 over time. When the concentration of human 20S was low (free Inhibitor 1), the mP was about 40 millipolarization units (mP). The fluorescence polarization at high concentrations of human 20S (bound Inhibitor 1) was about 110 mP.

### Example 4: Proteasome Competitive Binding Assay by Fluorescence Polarization

A non-labeled proteasome inhibitor (YU101) is serially diluted in DMSO then step diluted into 20 mM Tris, pH 8.0, 0.5 mM EDTA, 0.03% SDS buffer for a final assay concentration of 1% DMSO. This inhibitor is used as a competitive binder to human 20S proteasome (hu20S). The diluted inhibitor is incubated for 3 hours at room temperature with 10 nM hu20S in the presence of 2 nM fluorescein-labeled peptide ketoepoxide (Inhibitor 1). Fluorescence polarization values are then obtained.

When the concentration of the unlabeled proteasome inhibitor is low, the Inhibitor 1:hu20S complex is intact, and the fluorescence polarization is high. When the concentration of the unlabeled proteasome inhibitor is high, the Inhibitor 1:hu20S complex does not form, yielding low polarization values. In the specific case where irreversible inhibitors are used, the labeled and unlabeled inhibitors must be added to the human 20S proteasome simultaneously. If both the inhibitor and the labeled compound are reversible, the fluorescent-compound:hu20S complex could be preformed, and the inhibitor could be added later which would then cause the fluorophore to release from the proteasome yielding a decrease in polarization.

### Example 5: Determination of Drug Occupancy Using FACS

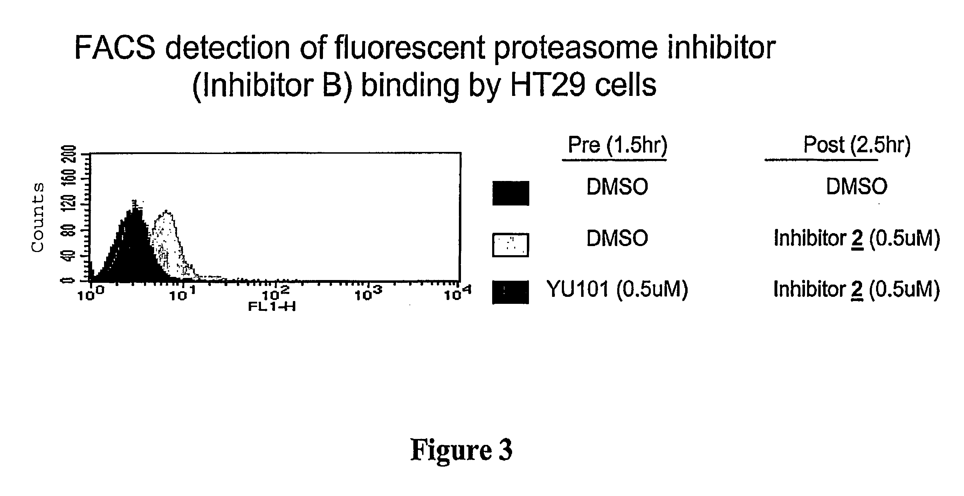

Intact cells (PBMC or dissociated tissues) were stained with Inhibitor 2 (with or without permeabilization), the samples were pre-treated with unlabeled YU101 to verify specificity of staining and binding was measured by flow cytometry (FACS) as shown in Figure 3.

### Example 6: Gel shift assay

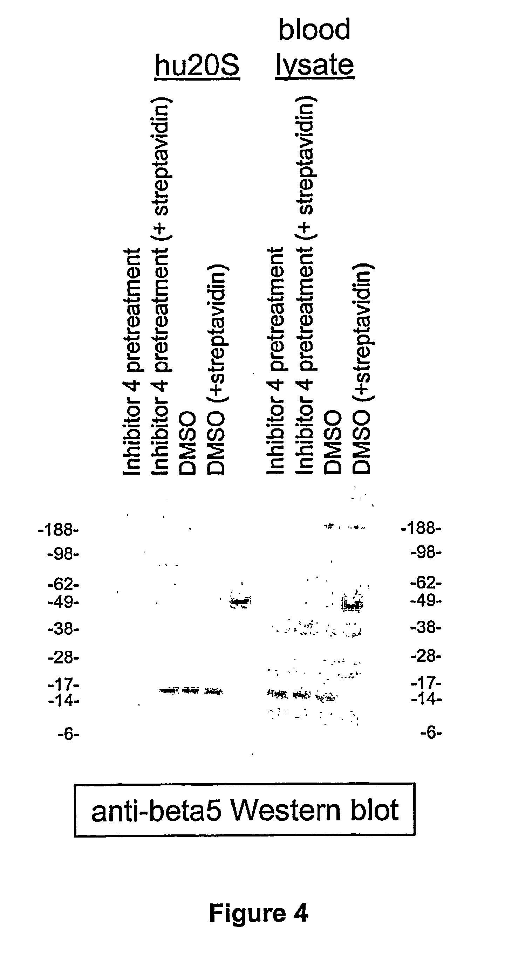

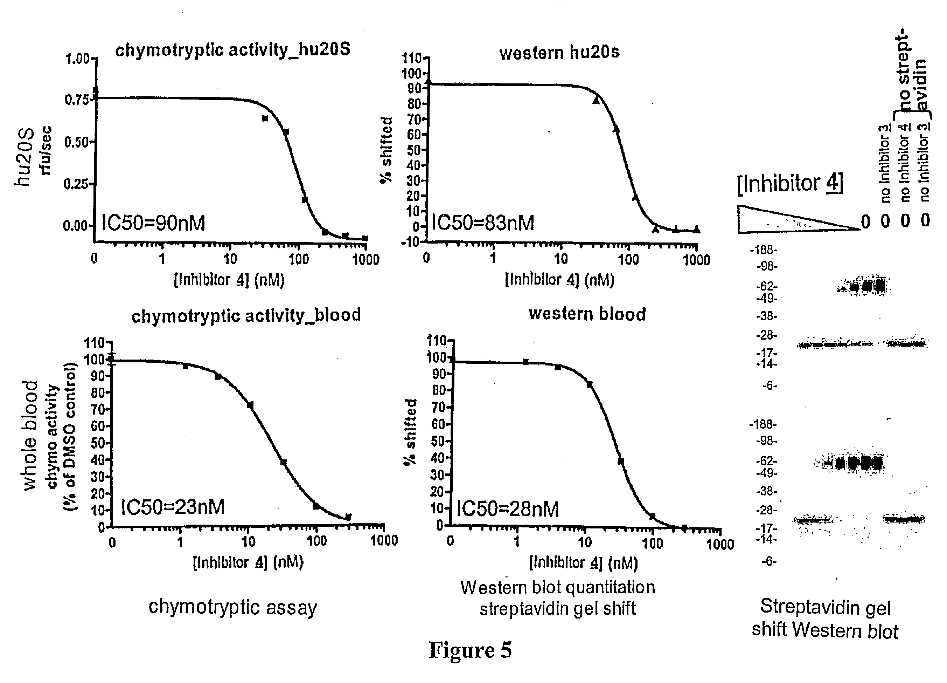

Human 20S (50 nM) or whole blood lysate (100 mg/mL) was pretreated with either DMSO or Inhibitor **4** (200 nM) for 1 h. Inhibitor 3 (1 µM) was then added. After 1 h, unbound Inhibitor **3** was removed using a spin desalting column and the resulting solution was diluted (1:10) in 1% SDS and heated at 90°C to denature the proteins. The solution was cooled to room temperature and streptavidin (±1 µg/15 µL) was added. The inhibitor/streptavidin solution was then added to NuPAGE sample buffer at room temperature (not boiled). The sample was then loaded on NuPAGE gel and run at 150 V in a refrigerator at approximately 4 °C. The Western blot was then probed with anti-beta5 rabbit polyclonal antibody (available from AffinityBioreagents) as shown in Figures **4** and 5. The shift in gel mobility demonstrates β5 binding site availability to Inhibitor **3.** Comparison of the Inhibitor **4** dose response between the gel shift assay and chymotryptic substrate cleavage assay demonstrates that the two assays yield comparable IC₅₀ values (Figure 5).

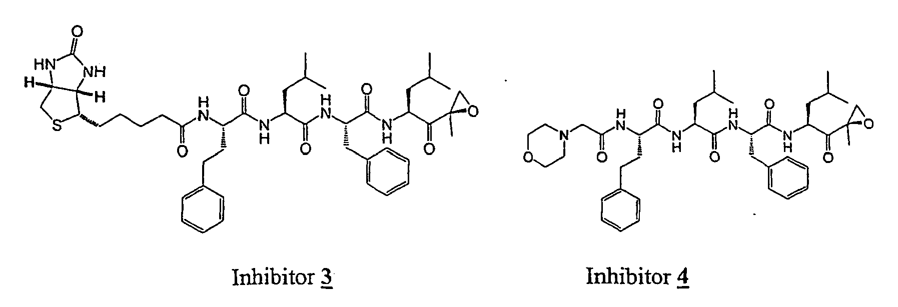

### Example 7: ELISA assay

Human whole blood or PBMC samples were treated with 0.1 nM to 1 µM of proteasome Inhibitor 4. The samples were then washed with phosphate-buffered saline (PBS) and lysed in hypotonic buffer (20 mM Tris pH 8, 5 mM EDTA) (Tris-HCl and EDTA are available from Teknova, Inc., Hollister, CA). Cellular debris was removed by centrifugation at 14,000 rpm in a microfuge (4 °C) for 2 min. The supernatant was transferred to a fresh tube, snap frozen in liquid nitrogen and stored at -80 °C. After thawing on ice, the samples (30 µl for assays run in triplicate) were treated with 500 nM of Inhibitor 3 for 1 hr at room temperature. Following treatment with Inhibitor 3, the lysate was denatured by addition of seven volumes of 1% SDS (210 µl) (available from Bio-Rad, Hercules, CA) and heating at 99 °C with vigorous shaking for 5 min. The sample was allowed to cool and two volumes (60 µl) of 10% Triton X-100 (available from Bio-Rad, Hercules, CA) was added.

Streptavidin sepharose beads (6.5 µl/well) (available from Amersham Biosciences, Piscataway, NJ), were washed three times with 1 ml PBS (available from Mediatech, Inc., Herndon, VA) in a microcentrifuge tube. The beads were resuspended in ELISA wash/block buffer (PBS + 0.1 % Tween 20+1% bovine serum albumin; 20 µl/well) and transferred to the wells of a 96 well filter plate (BSA is available from Sigma, St. Louis, MO; Tween is available from Calbiochem, San Diego, CA). Denatured whole blood or PBMC lysates that were treated with Inhibitor 3 were added to the filter plate wells containing the streptavidin sepharose beads (each sample assayed in triplicate) and incubated for 1 hr at room temperature with shaking (MultiScreen-DV Opaque Plates with low protein binding durapore membrane; available from Millipore, Billerica, MA). The unbound material was removed by gentle filtration and the beads were washed six times with ELISA wash/block buffer (200µl each).

Primary antibody to human 20S proteasome subunit β5 (rabbit polyclonal antibody; available from Biomol, Plymouth Meeting, PA) or human 20S immunoproteasome subunit LMP7 (rabbit polyclonal antibody; available from Affinity BioReagents, Golden, CO) was diluted 1:1000 in ELISA wash/block buffer, added to the beads (100 µl/well), and incubated for 1 hr at room temperature on an orbital shaker. The beads were washed six times with ELISA wash/block buffer with gentle filtration. Secondary antibody treatment (1:5000) and washing are as described for primary antibody (goat anti-rabbit antibody-HRP conjugate; available from Biosource, Camarillo, CA). The beads were then resuspended in 100 µl chemiluminescent detection reagent (Super Signal Pico Chemiluminescent Substrate™; available from Pierce, Rockford, IL) and luminescence was read on a Tecan plate reader.

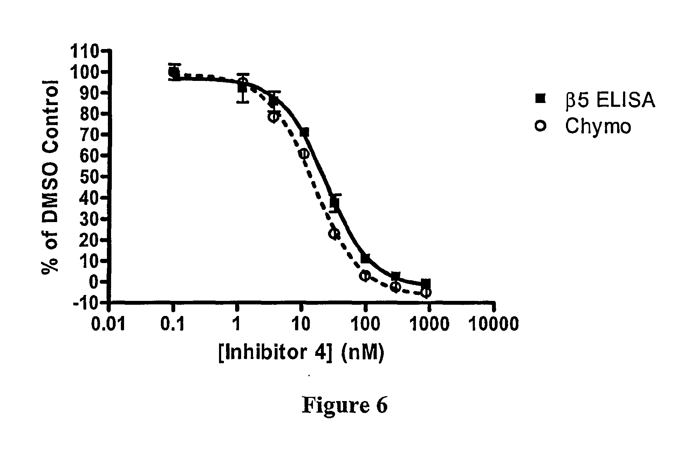

Occupation of the active sites of the proteasome with the peptide epoxyketone inhibitor results in both a decrease in chymotryptic-like catalytic activity (e.g., see Example 2) and a decrease in binding of the biotinylated probe (Inhibitor **3**). A comparison of the results with whole blood for Inhibitor 3 binding β5 ELISA and the conventional chymotryptic-like proteasome activity assay (LLVY-AMC substrate) is shown in Figure 6. These data suggest that the ELISA-based assay using the biontinylated probe accurately reflects the inhibitory activity of Inhibitor **4**.

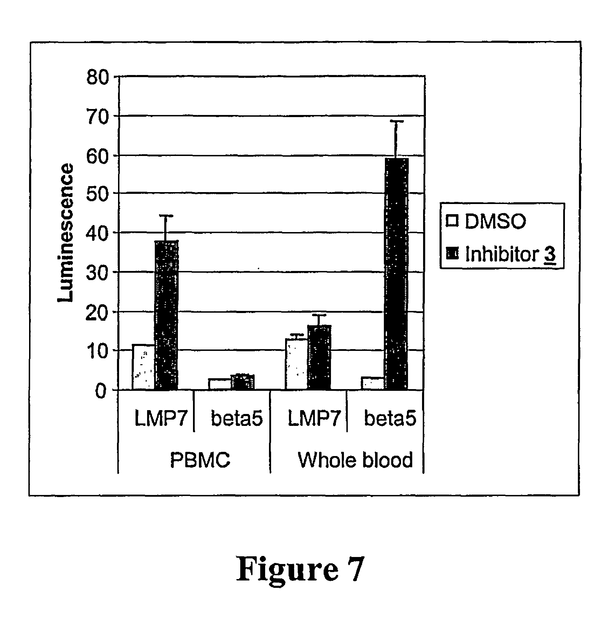

An exemplary feature of the ELISA-based PD assay is that it permits differentiation between constitutive proteasome inhibition (β5) and immunoproteasome inhibition (LMP7) because it utilizes subunit-specific antibodies. The ability of the ELISA-based assay to distinguish between constitutive proteasome and immunoproteasome is illustrated in Figure 7 which shows that the β5 antibody detects binding in whole blood but not PBMC while the LMP7 antibody detects binding in PBMC but not whole blood.

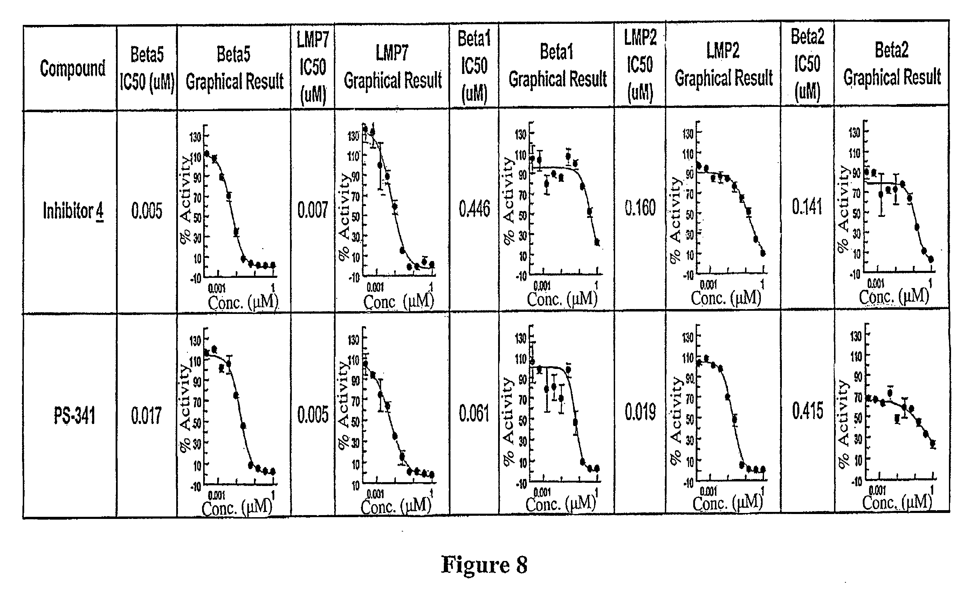

Utilizing a different active site probe (Inhibitor **5**) expands the utility of the ELISA-based assay for measuring the occupation of multiple constitutive (β5, (β1, β2) and immunoproteasome (LMP7, LMP2) active sites (Figure 8) in 8226 multiple myeloma cell line that co-expresses both forms of proteasome. The expanded active site assay can be used to measure relative inhibitor selectivity both between the immuno- and constitutive proteasomes as well as among the three active sites of each proteasome. In addition, the ELISA-based assay is able to determine the potency and selectivity of other classes of proteasome inhibitors, including the peptide boronic acid-based inhibitors (e.g., PS-341) (Figure 8).

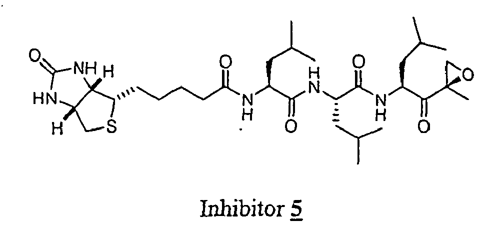

To conduct a pharmacodynamic evaluation of an inhibitor, whole blood and PBMC samples are collected prior to dosing and at multiple time-points after dosing. Lysates are prepared and protein concentration assays performed to normalize for total protein in each lysate. The level of inhibitor binding to β5 and LMP7 subunits in whole blood and PBMC, respectively, is determined by the streptavidin-capture ELISA described above. Standard curves with purified constitutive proteasome and immunoproteasome are utilized to ensure assay linearity/dynamic range and to convert the chemiluminescence signal to an absolute amount (µg) of subunit bound. To determine the level of proteasome inhibition for a given patient, the amount of β5 or LMP7 detected in the post-dose sample is compared to the pre-dose sample. Proteasome inhibition is determined after a single dose or after a cycle of dosing, or is used to monitor inhibition shortly after dosing as well as to monitor recovery of proteasome activity after a course of dosing.

### Example 8: Synthesis of Tagged Inhibitor 6

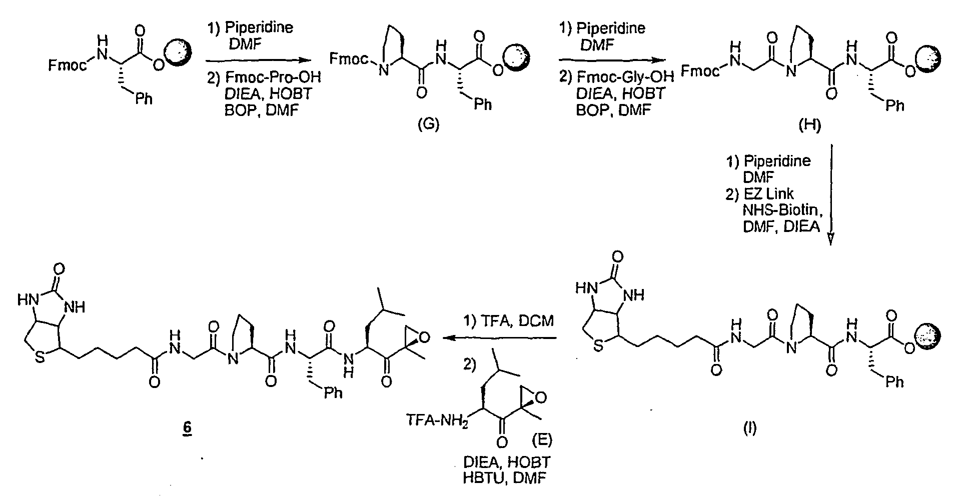

### Synthesis of (G)

To Fmoc-Phe-Wang resin (4.0 mmol, 5.0 g) was added a mixture of 20% piperidine/DMF (50 mL). The heterogeneous mixture was allowed to shake for 20 minutes. The mixture was then filtered and the resin washed with DMF (100 mL), MeOH (100 mL), and DCM (100 mL) and allowed to air dry. The resin was then subjected to the reaction conditions a second time to provide the resin bound primary amine. To the resin bound amine was added, in order, Fmoc-Pro-OH (7.9 mmol, 2.82 g), DEEA (13.3 mmol, 2.23 mL), and HOBT (8.10 mmol, 1.24 g). To this mixture BOP (7.98 mmol, 3.53 g) diluted in DMF (40 mL) was added slowly and the reaction mixture was allowed to shake overnight. The reaction mixture was then filtered and the resin washed with DMF (150 mL), MeOH (150 mL), DCM (150 mL), and allowed to air dry, to yield (G).

### Synthesis of (H)

To (A) (4.0 mmol) was added a mixture of 20% piperidine/DMF (50 mL) and the heterogeneous mixture was allowed to shake for 20 minutes. The mixuture was filtered and the resin washed with DMF (100 mL), MeOH (100 mL), and DCM (100 mL) and allowed to air dry. The resin was then subjected to the reaction conditions a second time to yield the resin bound primary amine. To the resin bound amine was added, in order,
H (7.9 mmol, 2.34 g), DIEA (13.3 mmol, 2.23 mL), and HOBT (8.10 mmol, 1.24 g). To the this mixture was slowly added BOP (7.98 mmol, 3.53 g) diluted in DMF (40 mL) and the reaction mixture was allowed to shake overnight. The reaction mixture was filtered and the resin washed with DMF (150 mL), MeOH (150 mL),and DCM (150 mL), and allowed to air dry, to yield (H).

### Synthesis of (1)

To (I) (0.125 mmol, 0.1 g) was added 50% TFA/DCM (2 mL) and the mixture was allowed to shake for 20 minutes during which time the resin turned purple. The mixture was filtered, the resin washed with DCM (10 mL), and the volatiles were removed under reduced pressure. The resulting oil was diluted with DCM (10 mL) and evaporated a total of three times to provide the resin bound amine. To the resin bound amine was added EZ Link NHS-Biotin (0.25 mmol, 0.085 g) followed by DMF (2 mL) and DIEA (0.5 mmol, 0.087 mL) and the reaction mixture was allowed to shake overnight. The reaction mixture was then filtered and the resin washed with DMF (150 mL), MeOH (150 mL), and DCM (150 mL), and allowed to air dry, to yield (I).

### Synthesis of Target Inhibitor 6

To (I) (0.125 mmol) was added 50% TFA/DCM (2 mL), and the reaction was allowed to shake for 20 minutes during which time the resin turned purple. The reaction was then filtered and the resin washed with DCM (10 mL).. The volatiles were removed under reduced pressure and the resulting oil was diluted with DCM (10 mL) and evaporated a total of three times to provide the intermediate carboxylic acid. To a stirred solution of (E) [see: Bioorg. Med. Chem. Letter 1999, 9, 2283-88] (19 mg, 0.11 mmol) in DMF (2 mL) was added the intermediate carboxylic acid (0.125 mmol), DIEA (2.9 mmol, 0.5 mL), HOBT (0.2 mmol, 0.032g), and HBTU (0.23 mmol, 0.087 g) and the mixture was stirred at room temperature overnight. The reaction was then diluted with brine (15 mL) and extracted with EtOAc. The organic layer was washed with water, sat. NaHCO₃, and brine and dried over anhydrous MgSO₄. The MgSO₄ was removed by filtration and the volatiles removed under reduced pressure. The crude material was purified by flash chromatography using 20 to 40% EtOAc/HeXane as the eluent, to afford Inhibitor **6**.

### Equivalents

Those skilled in the art will recognize, or be able to ascertain using no more than routine experimentation, numerous equivalents to the compounds and methods of use thereof described herein. Such equivalents are considered to be within the scope of this invention and are covered by the following claims.

All of the above-cited references and publications are hereby incorporated by reference.

The invention furthermore comprises the following items:
1. A compound having a structure of formula (I) or a pharmaceutically acceptable salt thereof, 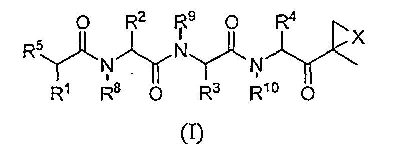
   - X: is selected from O, NH, and N-C₁₋₆alkyl;
   - R¹,R², R³, and R⁴: are each independently selected from C₁₋₆alkyl, C₁₋₆hydroxyalkyl, C₁₋₆alkoxyalkyl, aiyl, and C₁₋₆aralkyl, any of which is optionally substituted with one or more of amide, amine, carboxylic acid (or a salt thereof), ester, thiol, or thioether substituents;
   - R⁵: is N(R⁶)R⁷;
   - R⁶: is selected from hydrogen, OH, and C₁₋₆alkyl;
   - R⁷: comprises a detectable label;
   - R⁸,R⁹, and R¹⁰: are independently selected from hydrogen and C₁₋₆alkyl;
   provided that when R³ is C₁₋₆hydroxyalkyl, then R⁶ is hydrogen.
2. A compound of item 1, wherein R⁷ comprises a covalently conjugated moiety selected from a fluorescent moiety, a radioactive isotope-containing moiety, biotin, and a moiety that selectively binds to an antibody.
3. A compound of item 2, wherein X is O.
4. A compound of item 3, wherein R¹, R², R³, and R⁴ are each independently selected from C₁₋₆alkyl, C₁₋₆hydroxyalkyl, and C₁₋₆aralkyl.
5. A compound of item 4, wherein R¹, and R³ are each independently C₁₋₆aralkyl and R² and R⁴ are each independently C₁₋₆alkyl.
6. A compound of item 5, wherein R² and R⁴ are both isobutyl, R¹ is phenylethyl, and R³ is phenylmethyl.
7. A compound of item 6, wherein R⁶ is selected from hydrogen and C₁₋₆alkyl.
8. A compound of item 7, wherein R is hydrogen.
9. A compound of item 8, wherein R⁷ comprises a fluorescent moiety that is an amine-reactive dye.
10. A compound of item 8, wherein R⁷ comprises a moiety that selectively binds to an antibody.
11. A compound of item 8, wherein R⁷ comprises biotin.
12. A compound of item 8, wherein R⁷ comprises a radioactive isotope-containing moiety selected from C₁₋₆alkyl, C₁₋₆hydroxyalkyl, C₁₋₆alkoxyalkyl, aryl, and C₁₋₆aralkyl, and R⁷ includes at least one radioactive label selected from ³H, ¹¹C, ¹⁴C, ¹³N, ¹⁵O, and ¹²⁵I.
13. A compound of item 8, wherein R comprises an amino acid or peptide moiety that includes at least one radioactive moiety selected from ³H, ¹¹C, ¹⁴C, ¹³N_{,} ¹⁵O, and **¹²⁵I.**
14. A compound having a structure of formula II or a pharmaceutically acceptable salt thereof, 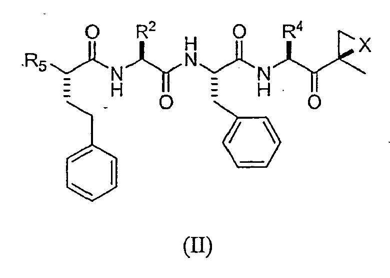 wherein,
   - X: is selected from O, NH, and N-C₁₋₆alkyl;
   - R²and R⁴: are each independently selected from C₁₋₆alkyl, C₁₋₆hydroxyalkyl, C₁₋₆alkoxyalkyl, aryl, and C₁₋₆aralkyl, any of which is optionally substituted with one or more of amide, amine, carboxylic acid (or a salt thereof), ester, thiol, or thioether substituents;
   - R⁵: is N(R⁶)R⁷;

   - R⁶: is selected from hydrogen, OH, and C₁₋₆alkyl;
   - R⁷: comprises a detectable label.
15. A compound of item 14, wherein R⁷ comprises a covalently conjugated moiety selected from a fluorescent moiety, a radioactive isotope-containing moiety, biotin, and a moiety that selectively binds to an antibody.
16. A compound of item 15, wherein X is O.
17. A compound of item 16, wherein R¹, R², R³, and R⁴ are each independently selected from C₁₋₆alkyl, C₁₋₆hydroxyalkyl, and C₁₋₆aralkyl.
18. A compound of item 17, wherein R¹, and R³ are each independently C₁₋₆aralkyl and R² and R⁴ are each independently C₁₋₆alkyl.
19. A compound of item 18, wherein R and R⁴ are both isobutyl, R¹ is phenylethyl, and R³ is phenylmethyl.
20. A compound of item 19, wherein R⁶ is selected from hydrogen and C₁₋₆alkyl.
21. A compound of item 20, wherein R⁶ is hydrogen.
22. A compound of item 21, wherein R⁷ comprises a fluorescent moiety that is an amine-reactive dye.
23. A compound of item 21, wherein R⁷ comprises a moiety that selectively binds to an antibody.
24. A compound of item 21, wherein R⁷ comprises biotin.
25. A compound of item 21, wherein R⁷ comprises a radioactive isotope-containing moiety selected from C₁₋₆alkyl, C₁₋₆bydroxyalkyl, C₁₋₆alkoxyalkyl, aryl, and C₁₋₆aralkyl, and R⁷ includes at least one radioactive label selected from ³H, ¹¹C, ¹⁴C, ¹³N, ¹⁵O, and ¹²⁵I.
26. A compound of item 21, wherein R⁷ comprises an amino acid or peptide moiety that includes at least one radioactive label selected from ³H, ¹¹C, ¹⁴C, ¹³N, ¹⁵O, and ¹²⁵I_{.}
27. A compound having a structure of formula III or a pharmaceutically acceptable salt thereof, 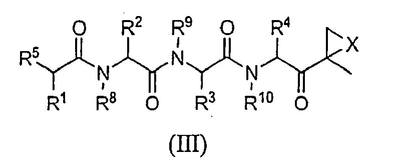
   - X: is selected from O, NH, and N-C₁₋₆alky;
   - R¹,R², R³, and R⁴: are each independently selected from hydrogen, C1-6alkyl, C₁₋₆hydroxyalkyl, C₁₋₆alkoxyalkyl, aryl, and C₁₋₆aralkyl, any of which is optionally substituted with one or more of amide, amine, carboxylic acid, ester, thiol, or thioether substituents;
   - R⁵: is N(R⁶)R⁷;
   - R⁶: is selected from hydrogen, OH, and C₁₋₆alkyl;
   - R⁷: comprises a detectable label; and
   - R⁸,R⁹, and R¹⁰: are independently selected from hydrogen and C₁₋₆alkl; or R² and R⁸, R³ and R⁹, or R⁴ and R¹⁰ are together C₂₋₅alkyl.
28. A compound of item 27, wherein R⁷ comprises a covalently conjugated moiety selected from a fluorescent moiety, a radioactive isotope-containing moiety, biotin, and a moiety that selectively binds to an antibody.
29. A compound of item 28, wherein X is O.
30. A compound of item 29, wherein R¹, R³, and R⁴ are each independently selected from hydrogen, C₁₋₆alkyl, and C₁₋₆aralkyl.
31. A compound of item 30, wherein R¹ is hydrogen, R³ is selected from C₁₋₆alkyl and C₁₋₆aralkyl, and R⁴ is C₁₋₆alkyl.
32. A compound of item 31, wherein R³ is selected from isobutyl and phenylmethyl and R⁴ is isobutyl.
33. A compound of item 32, wherein R² and R⁸ together are C₃alkyl, thereby forming a ring.
34. A compound of item 32, wherein R is selected from C₁₋₆alkyl and C₁₋₆aralkyl.
35. A compound of item 34, wherein R² is isobutyl and R⁸, R⁹, and R¹⁰ are all hydrogen.
36. A compound of item 35, wherein R⁶ is selected from hydrogen and C₁₋₆alkyl.
37. A compound of item 36, wherein R⁶ is hydrogen.
38. A compound of item 37, wherein R⁷ comprises a fluorescent moiety that is an amine-reactive dye.
39. A compound of item 37, wherein R⁷ comprises a moiety that selectively binds to an antibody.
40. A compound of item 37, wherein R⁷ comprises biotin.
41. A compound of item 37, wherein R⁷ comprises a radioactive isotope-containing moiety selected from C₁₋₆alkyl, C₁₋₆hydroxyalkyl, C₁₋₆alkoxyalkyl, aryl, and C₁₋₆aralkyl, and R⁷ includes at least one radioactive label selected from ³H, ¹¹C, ¹⁴C, ¹³N, ¹⁵O, and ¹²⁵I_{.}
42. A compound of item 37, wherein R⁷ comprises an amino acid or peptide moiety that includes at least one radioactive label selected from ³H, ¹¹C, ¹⁴C, ¹³N, ¹⁵O, and ¹²⁵I_{.}
43. A pharmaceutical composition comprising a compound of any one of item 1 to 42 and a pharmaceutically acceptable carrier.
44. A method of inhibiting an N-terminal nucleophile hydrolase, wherein said inhibitor inhibits a chymotrypsin-like activity of said 20S proteasome when said inhibitor is present at concentrations below about 5 µM, and does not inhibit trypsin-like activity or PGPH activity of said 20S proteasome when said inhibitor is present at concentrations below about 5 µM.
45. A method for determining the biodistribution of a compound of item 9 or 38, comprising
   (a) administering the compound to a biological sample; and
   (b) subjecting the biological sample to fluorescence microscopy, whole animal imaging, or fluorescence polarization.
46. A method of item 45, wherein the biological sample is selected from purified 20S, purified 26S, patient tissue, cells, and whole animals.
47. A method for identifying and characterizing the binding characteristics of a potential proteasome inhibitor, comprising quantitating the available 20S proteasome enzymatic active sites before and after administration of a potential or known proteasome inhibitor using a compound of item 1 or 27.
48. A method for determining the drug occupancy of the proteasome 20S enzymatic active site, comprising
   (a) determining the amount of a compound of item 1 or 27 that binds to a test biological sample obtained from a mammal treated with a proteasome inhibitor;
   (b) comparing the amount of the compound in (a) to the amount of the compound that binds to a reference biological sample obtained from a mammal that was not treated with an inhibitor.
49. A method of item 48, wherein the biological sample is selected from blood, urine, and organ or tissue samples.
50. A method of item 49, wherein the biological sample is a blood sample selected from whole blood, PBMC, and white blood cells.
51. A method of item 49, wherein the biological sample is selected from a tumor biopsy, colon biopsy, skin biopsy, synovial fluid, bronchial fluid, muscle cells, and bone marrow/blood cell precursors.
52. A method of item 48, wherein the compound of item 1 or 31 comprises a radioactive isotope-containing moiety.
53. A method of item 48, wherein the compound of item 1 or 31 comprises an affinity tag.
54. A method of item 53, wherein the affinity tag is biotin.
55. A method for determining the activity of a proteasome inhibitor, comprising:
   (a) obtaining a biological sample that has been treated with a proteasome inhibitor;
   (b) separating inhibitor-bound proteasome subunits from unbound proteasome subunits;
   (c) determining the amount of inhibitor-bound proteasome subunits, unbound proteasome subunits, or both, wherein a change in the amount of inhibitor bound proteasome subunits is indicative of proteasome inhibitor activity.
56. The method of item 55, further comprising contacting the biological sample with a detectable label that binds to a proteasome subunit.
57. The method of item 56, wherein the detectable label is a labeled proteasome inhibitor.
58. The method of item 57, wherein the proteasome inhibitor is labeled with one of the following: a fluorescent moiety, a chemiluminescent moiety, a paramagnetic contrast agent, a metal chelate, a radioactive isotope-containing moiety, biotin, or a moiety that selectively binds to an antibody.
59. The method of item 58, wherein the proteasome inhibitor is labeled with biotin.
60. The method of item 57, wherein the proteasome inhibitor is a compound of item 1 or 31.
61. The method of item 56, wherein unbound proteasome subunits are separated from bound proteasome subunits using the detectable label.
62. The method of item 55 or 56, wherein the unbound proteasome subunits are separated from bound proteasome subunits by size separation.
63. The method of item 62, wherein the size separation is gel electrophoresis.
64. The method of item 62, wherein the size separation is column chromatography.
65. The method of item 55, further comprising contacting the sample with at least one antibody that binds to a proteasome subunit.
66. The method of item 65, wherein the antibody binds to a subunit of the constitutive proteasome.
67. The method of item 66, wherein the antibody binds to the β1, β2, or β5 subunit of the constitutive proteasome.
68. The method of item 65, wherein the antibody binds to a subunit of the immunoproteasome.
69. The method of item 68, wherein the antibody binds to the LMP7, LMP2, or MECL1 subunit of the immunoproteasome.
70. The method of item 65, further comprising determining the amount of proteasome subunits bound to the antibody.
71. The method of item 55, wherein the biological sample is obtained from a mammal that has been treated with a proteasome inhibitor.
72. The method of item 71, wherein the mammal is a human.
73. The method of item 55 or 71, further comprising comparing the amount of inhibitor-bound proteasome subunits, unbound proteasome subunits, or both, in the biological sample to a control.
74. The method of item 73, wherein the control is a biological sample from a mammal that was not treated with a proteasome inhibitor.
75. The method of item 73, wherein the control is a biological sample from the mammal prior to treatment with the proteasome inhibitor.
76. The method of item 55, wherein the biological sample comprises at least one of the following: blood, urine, organ biopsy, or tissue biopsy.
77. The method of item 76, wherein the biological sample comprises at least one of the following: whole blood, PBMC, or white blood cells.
78. The method of item 76, wherein the biological sample comprises at least one of the following: a tumor biopsy, a colon biopsy, a skin biopsy, synovial fluid, bronchial fluid, muscle cells, or bone marrow/blood cell precursors.
79. The method of item 71, further comprising obtaining two or more biological samples from said mammal at specified times after administration of the proteasome inhibitor to monitor the pharmacodynamic drug action of the proteasome inhibitor.
80. A method for determining the activity of a proteasome inhibitor, comprising:
   (a) obtaining a biological sample from a mammal treated with a proteasome inhibitor;
   (b) contacting the sample with a compound having a structure of formula (I) or formula (III) or a pharmaceutically acceptable salt thereof, 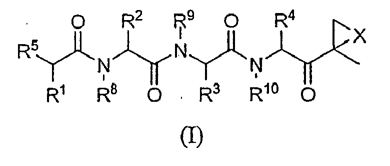 wherein,
      - X: is selected from O, NH, and N-C₁₋₆alkyl;
      - R¹,R², R³, and R⁴: are each independently selected from C₁₋₆alkyl, C₁₋₆hydroxyalkyl, C₁₋₆alkoxyalkyl, aryl, and C₁₋₆aralkyl, any of which is optionally substituted with one or more of amide, amine, carboxylic acid (or a salt thereof), ester (including C₁₋₅ alkyl ester and aryl ester), thiol, or thioether substituents;
      - R⁵: is N(R⁶)R⁷;
      - R⁶: is selected from hydrogen, OH, and C₁₋₆alkyl; and
      - R⁷: comprises a detectable label;
      - R⁸R⁹, and R¹⁰: are independently selected from hydrogen and C₁₋₆alkyl;

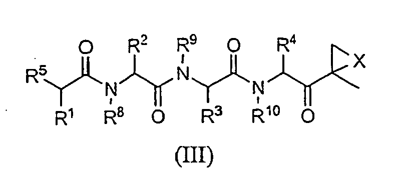

      - X: is selected from O, NH, and N-C₁₋₆alky;
      - R¹, R², R³, and R⁴: are each independently selected from hydrogen, C₁₋₆alkyl, C₁₋₆hydroxyalkyl, C₁₋₆alkoxyalkyl, aryl, and C₁₋₆aralkyl, any of which is optionally substituted with one or more of amide, amine, carboxylic acid, ester, thiol, or thioether substituents;
      - R⁵: is N(R⁶)R⁷;
      - R⁶: is selected from hydrogen, OH, and C₁₋₆alkyl;
      - R⁷: comprises a detectable label; wherein the detectable label may include both a label portion and a linker portion; and
      - R⁸,R⁹, and R¹⁰: are independently selected from hydrogen and C₁₋₆alkyl; or R² and R⁸, R³ and R⁹, or R⁴ and R¹⁰ are together C₂₋₅alkyl;
   (c) separating proteasome subunits that bound to a compound of formula (I) or formula (III) from unbound proteasome subunits; and
   (d) contacting the sample with an antibody that binds to a subunit of the constitutive proteasome or a subunit of the immunoproteasome to determine the amount of proteasome subunits from the constitutive proteasome or immunoproteasome that bound to a compound of formula (I) or formula (III) as compared to a control sample from a mammal that was not treated with the proteasome inhibitor, wherein a decrease in the amount of proteasome subunits that bound to a compound of formula (I) or formula (III) as compared to the control is indicative of proteasome inhibitor activity.
81. The method of item 80, wherein R⁷ comprises a covalently conjugated moiety selected from a fluorescent moiety, a radioactive isotope-containing moiety, biotin, and a moiety that selectively binds to an antibody.
82. The method of item 81, wherein R⁷ comprises biotin.
83. The method of item 82, wherein the proteasome subunits that bound to a compound of formula (I) or formula (III) are separated from unbound proteasome subunits using streptavidin functionalized beads.
84. The method of item 80, wherein the sample is contacted with an antibody that binds to the β1, β2, or β5 subunit of the constitutive proteasome.
85. The method of item 80, wherein the sample is contacted with an antibody that binds to the LMP7, LMP2, or MECL1 subunit of the immunoproteasome.

## Claims

1. A method for determining the activity of a proteasome inhibitor, comprising:
(a) obtaining a biological sample that has been treated with a proteasome inhibitor;
(b) separating inhibitor-bound proteasome subunits from unbound proteasome subunits;
(c) determining the amount of inhibitor-bound proteasome subunits, unbound proteasome subunits, or both, wherein a change in the amount of inhibitor bound proteasome subunits is indicative of proteasome inhibitor activity.

2. The method of claim 1, further comprising contacting the biological sample with a detectable label that binds to a proteasome subunit.

3. The method of claim 2, wherein the detectable label is a labeled proteasome inhibitor.

4. The method of claim 3, wherein the proteasome inhibitor is labeled with one of the following: a fluorescent moiety, a chemiluminescent moiety, a paramagnetic contrast agent, a metal chelate, a radioactive isotope-containing moiety, biotin, or a moiety that selectively binds to an antibody.

5. The method of claim 4, wherein the proteasome inhibitor is labeled with biotin.

6. The method of claim 3, wherein the proteasome inhibitor is a compound selected from
(i) a compound having a structure of formula (I) or a pharmaceutically acceptable salt thereof, 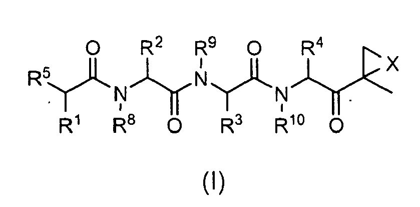 X is selected from O, NH, and N-C₁₋₆alkyl;
R¹, R², R³, and R⁴ are each independently selected from hydrogen, C₁₋
₆alkyl, C₁₋₆hydroxyalkyl, C₁₋₆alkoxyalkyl, aryl, and C₁₋₆aralkyl, any of which is optionally substituted with one or more of amide, amine, carboxylic acid (or a salt thereof), ester, thiol, or thioether substituents;
R⁵ is N(R⁶)R⁷;
R⁶ is selected from hydrogen, OH, and C₁₋₆alkyl;
R⁷ comprises a detectable label; and
R⁸, R⁹, and R¹⁰ are independently selected from hydrogen and C₁₋₆alkyl; provided that when R³ is C₁₋₆hydroxyalkyl, then R⁶ is hydrogen; or
(ii) a compound having a structure of formula (III) or a pharmaceutically acceptable salt thereof, 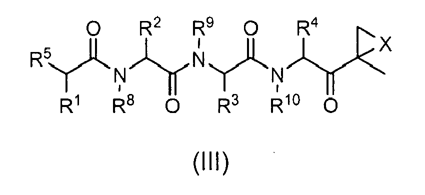 X is selected from O, NH, and N-C₁₋₆alkyl;
R¹ is hydrogen;
R² is selected from hydrogen, C₁₋₆alkyl, C₁₋₆hydroxyalkyl, C₁₋₆alkoxyalkyl, aryl, and C₁₋₆aralkyl, any of which is optionally substituted with one or more of amide, amine, carboxylic acid (or a salt thereof), ester, thiol, or thioether substituents;
R³ is selected from C₁₋₆alkyl and C₁₋₆aralkyl;
R⁴ is C₁₋₆alkyl;
R⁵ is N(R⁶)R⁷;
R⁶ is selected from hydrogen, OH, and C₁₋₆alkyl;
R⁷ comprises a detectable label; and
R⁸, R⁹, and R¹⁰ are independently selected from hydrogen and C₁₋₆alkyl.

7. The method of claim 2, wherein unbound proteasome subunits are separated from bound proteasome subunits using the detectable label.

8. The method of claim 1 or 2, wherein the unbound proteasome subunits are separated from bound proteasome subunits by size separation.

9. The method of claim 1, further comprising contacting the sample with at least one antibody that binds to a proteasome subunit.

10. The method of claim 9, wherein the antibody binds to a subunit of the constitutive proteasome.

11. The method of claim 10, wherein the antibody binds to the β1, β2, or β5 subunit of the constitutive proteasome.

12. The method of claim 9, wherein the antibody binds to a subunit of the immunoproteasome.

13. The method of claim 12, wherein the antibody binds to the LMP7, LMP2, or MECL1 subunit of the immunoproteasome.

14. The method of claim 9, further comprising determining the amount of proteasome subunits bound to the antibody.

15. The method of claim 1, wherein the biological sample is obtained from a mammal that has been treated with a proteasome inhibitor.

16. The method of claim 15, wherein the mammal is a human.

17. The method of claim 1 or 15, further comprising comparing the amount of inhibitor-bound proteasome subunits, unbound proteasome subunits, or both, in the biological sample to a control.

18. The method of claim 17, wherein the control is a biological sample from a mammal that was not treated with a proteasome inhibitor.

19. The method of claim 17, wherein the control is a biological sample from the mammal prior to treatment with the proteasome inhibitor.

20. The method of claim 1, wherein the biological sample comprises at least one of the following: blood, urine, organ biopsy, or tissue biopsy.

21. The method of claim 20, wherein the biological sample comprises at least one of the following: whole blood, PBMC, or white blood cells.

22. The method of claim 20, wherein the biological sample comprises at least one of the following: a tumor biopsy, a colon biopsy, a skin biopsy, synovial fluid, bronchial fluid, muscle cells, or bone marrow/blood cell precursors.

23. The method of claim 15, further comprising obtaining two or more biological samples from the mammal at specified times after administration of the proteasome inhibitor to monitor the pharmacodynamic drug action of the proteasome inhibitor.
